(19) **Europäisches Patentamt
European Patent Office
Office européen des brevets**

(11) **EP 4 564 361 B1**

(12) **EUROPEAN PATENT SPECIFICATION**

(45) Date of publication and mention
of the grant of the patent:
**29.07.2026 Bulletin 2026/31**

(21) Application number: **23734697.8**

(22) Date of filing: **12.06.2023**

(51) International Patent Classification (IPC):
***G16H 20/17*** (2018.01)     ***G16H 50/50*** (2018.01)

(52) Cooperative Patent Classification (CPC):
**G16H 20/17; G16H 50/50**

(86) International application number:
**PCT/ES2023/070385**

(87) International publication number:
**WO 2024/023376 (01.02.2024 Gazette 2024/05)**

(54) **METHOD FOR IMPROVING BLOOD GLUCOSE CONTROL OF A HYBRID CONTROLLER**

VERFAHREN ZUR VERBESSERUNG DER BLUTZUCKERKONTROLLE EINES
HYBRIDSTEUERGERÄTS

MÉTHODE POUR AMÉLIORER LA RÉGULATION DE LA GLYCÉMIE D'UN RÉGULATEUR
HYBRIDE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **28.07.2022 ES 202230693**

(43) Date of publication of application:
**04.06.2025 Bulletin 2025/23**

(73) Proprietor: **Universitat Politècnica de València
46022 Valencia (ES)**

(72) Inventors:
• **SALA MIRA, Iván**
46022 Valencia (ES)
• **GARCÍA GIL, Pedro José**
46022 Valencia (ES)
• **BONDÍA COMPANY, Jorge**
46022 Valencia (ES)
• **DIEZ RUANO, José Luís**
46022 Valencia (ES)

(74) Representative: **Pons IP
Glorieta Rubén Darío 4
28010 Madrid (ES)**

(56) References cited:
**AU-A1- 2013 309 425     US-A1- 2022 044 783**

• **BONDIA JORGE ET AL: "Insulin Estimation and Prediction: A Review of the Estimation and Prediction of Subcutaneous Insulin Pharmacokinetics in Closed-Loop Glucose Control", IEEE CONTROL SYSTEMS, IEEE, USA, vol. 38, no. 1, 1 February 2018 (2018-02-01), pages 47 - 66, XP011676166, ISSN: 1066-033X, [retrieved on 20180118], DOI: 10.1109/MCS.2017.2766312**
• **PRASHAR KRISHMA ET AL: "Robust Reduced-Order Model Based Postprandial Glucose Regulation in Type-1 Diabetes: An IMC Approach", 2021 SEVENTH INDIAN CONTROL CONFERENCE (ICC), IEEE, 20 December 2021 (2021-12-20), pages 254 - 258, XP034084757, DOI: 10.1109/ICC54714.2021.9703138**
• **RAMKISSOON CHARRISE MARY ET AL: "Detection and Control of Unannounced Exercise in the Artificial Pancreas Without Additional Physiological Signals", IEEE JOURNAL OF BIOMEDICAL AND HEALTH INFORMATICS, IEEE, PISCATAWAY, NJ, USA, vol. 24, no. 1, 1 January 2020 (2020-01-01), pages 259 - 267, XP011764444, ISSN: 2168-2194, [retrieved on 20200102], DOI: 10.1109/JBHI.2019.2898558**

**EP 4 564 361 B1**

• **ANONYMUOUS: "Internal model (motor control)", WIKIPEDIA, 14 May 2022 (2022-05-14), pages 1 - 2, XP093068090, Retrieved from the Internet <URL:https://en.wikipedia.org/w/index. php?title=Internal_model_(motor_control)& oldid=1087779190> [retrieved on 20230727], DOI: 10.1016/S0959-4388(99)00028-8**

**Description**

**OBJECT OF THE INVENTION**

The present invention is framed in the field of medical procedures and devices, and more particularly, in the diabetes care.

An object of the present invention is a method for improving blood glucose control of a hybrid controller allowing to eliminate the need for meal and exercise announcement by substituting patient-initiated meal boluses of said hybrid controller by an automatic insulin correction signal without retuning of said hybrid controller, and by incorporating rescue carbohydrates suggestion for hypoglycemia mitigation.

Another object of the invention is an add-on module for incorporating in a hybrid artificial pancreas, which incorporates a modified Internal Model Control that removes meal and exercise announcements.

Another object of the invention is an artificial pancreas system which includes the add-on module for carrying out the method of the invention.

**BACKGROUND OF THE INVENTION**

Closed-loop glucose control, i.e. artificial pancreas, outperforms other insulin therapies treating type 1 diabetes, such as multiple daily injections insulin therapy or sensor-augmented pump.

This technology reduces time in high glucose values (hyperglycemia) and associated risks such as retinopathy, neuropathy, or cardiovascular disease. The artificial pancreas also reduces time in low blood glucose values (hypoglycemia), with critical short-term complications (e.g., cognitive dysfunction, seizures, or coma in severe cases), and enhances the quality of life, by reducing anxiety or insomnia among others.

However, external disturbances, namely, meals and exercise, challenge the performance of artificial pancreas systems.

On the one hand, glucose ingested from meals reaches the bloodstream faster than subcutaneous insulin. Slow subcutaneous insulin absorption and sensor lag delay the insulin action, leading to sizeable postprandial glucose excursions. Insulin stacked in subcutaneous depots continues to be absorbed even after the meal absorption, which may also cause hypoglycemia.

On the other hand, exercise is beneficial for managing type 1 diabetes: it improves insulin sensitivity, reduces cardiovascular risks, improves bones health, etc. However, exercise unbalances glucose homeostasis and may cause hypoglycemia or hyperglycemia, depending on the kind of exercise, its duration, and its intensity. Low-to-moderate aerobic exercise usually lowers glucose; the fear of subsequent hypoglycemia constitutes the main reason people with type 1 diabetes give up an active lifestyle.

Current artificial pancreas systems require patient intervention (they are "hybrid" systems) to counteract meals and exercise. Insulin boluses at mealtime in hybrid artificial pancreas effectively reduce postprandial glucose excursions, but subjects need to timely provide an accurate estimation of the ingested carbohydrate to the system. This estimation is challenging. Then, estimation errors, bolusing delays, or omissions frequently degrade the performance achieved by the system.

Hybrid artificial pancreas systems usually modify glucose reference or basal profile to reduce the impact of exercise, which requires subjects to announce exercise time or intensity even with anticipation.

Some authors have proposed adding carbohydrate suggestions to cope with unannounced exercise events but their proposals still need meal announcements.

Other authors have proposed a meal-detector-based control to remove the meal announcement, but without considering exercise.

Therefore, an improvement is required in current artificial pancreas so as to allow to avoid the need for user intervention in view of meal and exercise events.

Publication AU2013309425A1 is considered as closest prior art because it discloses a closed loop control for insulin administration using a Laplace variable. But it fails to disclose all mathematical details of claim 1 of the present invention.

**DESCRIPTION OF THE INVENTION**

The present invention relates to a method for improving blood glucose control of a hybrid controller and is defined by the appended claims. The method of the invention allows to eliminate meal and exercise announcement by substituting patient-initiated meal boluses of said hybrid controller by an automatically generated insulin correction signal without retuning of said hybrid controller, and by incorporating rescue carbohydrates suggestion for hypoglycemia mitigation. The lack of need for retuning of the hybrid controller allows the incorporation of the method of the invention as an extra feature of a system already existing and extensively clinically validated, which can be configured to perform a fully automated mode (activating an add-on module implementing the method of the invention).

Another object of the invention is an add-on module based on an internal model control (IMC) that eliminates meal and exercise announcements from any hybrid controller that includes some restriction of the insulin-on board. The add-on module comprises a calculation unit configured to carry out the method for improving blood glucose control of the hybrid controller.

Another object of the invention is a new artificial pancreas system, which achieves good results in postprandial control. The artificial pancreas system comprises:

- a pump (3), for supplying insulin according to a coordinated control action ($u_c$);
- a continuous glucose monitor (2) for measuring the plasma glucose (G(t)) signal;
- a controller for determining the insulin level to be delivered, and
- the add-on module described.

Preferably, the module is implemented in an artificial pancreas comprising a controller incorporating a method for limitation of insulin-on-board by means of a safety layer acting on glucose reference so that a tunable upper limit of insulin-on-board is not violated.

In the art, the design of the control algorithm embedded in the hybrid artificial pancreas system, or "main controller", considers that the user will inform the system about meal intakes and exercise. In the absence of subject announcements, the main controller will likely perform unsatisfactorily.

The module implements an internal model control loop (IMC) that calculates a virtual signal $u_{IMC}(t)$ compensating for the discrepancy between the actual output and an output estimated by a nominal model. Then, a switching logic decomposes this virtual signal in a bolus-like insulin infusion ($u_{ins}(t)$), correcting the insulin given by the main controller, and rescue carbohydrates suggestions ($U_{resc}(t)$) to compensate for hyperglycemia and hypoglycemia, respectively.

The switching logic also makes more restrictive the tolerated insulin-on-board after suggesting a rescue carbohydrate intake.

The modification of the tolerated insulin-on-board is the only change the proposed module applies to the internal parameters of the main controller. Most of the hybrid systems constrain the insulin-on-board through gains or thresholds; hence the integration of the module with the main controller is immediate.

The IMC loop requires a glucose-insulin model M. Among the several control-oriented models that could be used in this approach, the Identifiable Virtual Patient (IVP) is preferable because of its structural simplicity and physiological interpretability.

The equations of the model are defined as follows,

$$\dot{I}_{SC}(t) = -\frac{1}{\tau_1} I_{SC}(t) + \frac{\kappa}{\tau_1 C_I} u_T(t) \qquad (1a)$$

$$\dot{I}_P(t) = -\frac{1}{\tau_2} I_P(t) + \frac{1}{\tau_2} I_{SC}(t) \qquad (1b)$$

$$\dot{I}_{EFF}(t) = -p_2 I_{EFF}(t) + p_2 S_I I_P(t) \qquad (1c)$$

$$\dot{d}_1(t) = A_g^{resc} \cdot u_{resc}(t) - \frac{d_1(t)}{\tau_{resc}} \qquad (1d)$$

$$\dot{d}_2(t) = \frac{1}{\tau_{resc}} \left( d_1(t) - d_2(t) \right) \qquad (1e)$$

$$\dot{G}(t) = -GEZI \cdot G(t) - I_{EFF}(t) \cdot G(t) + EGP + \frac{d_2(t)}{V_g \tau_{resc}} \qquad (1f)$$

where $I_{SC}(t)$ and $I_P(t)$ are the subcutaneous and plasma insulin concentrations (μU/mL), respectively. State $I_{EFF}(t)$ represents the insulin effect (min⁻¹), and G(t) is the plasma glucose concentration (mg/dL).

The known inputs of the model are the subcutaneous insulin infusion $u_T(t)$ ($\mu$U/min) and the rescue carbohydrate suggestion $u_{resc}(t)$ (mg/min).

A two-compartment model, with the glucose masses (mg) $d_1(t)$, $d_2(t)$ as states, models the rescue carbohydrates absorption. The parameters $\tau_1$ and $\tau_2$ (min) stand for the insulin absorption time constants, and $p_2$ is the kinetic rate for insulin action (min$^{-1}$). Parameter $C_I$ denotes the insulin clearance (mL/min), $S_I$ represents the insulin sensitivity (mL/$\mu$U), EGP is the hepatic glucose production (mg/dL/min), GEZI corresponds to the glucose effectiveness at zero insulin (min$^{-1}$).

Parameter $\tau_{resc}$ is the time to the peak absorption of the rescue carbohydrate, and $A_g^{resc}$ is the carbohydrate bioavailability. Finally, $\kappa = 60 \cdot 10^{-6}$ is a factor that converts the units of $u_T(t)$ from $\mu$U/min into U/h.

Any other factor affecting glucose (meals aside rescue carbohydrates suggested by the system and exercise among others) will correspond to output disturbances.

Independent of the model M selected, the method for improving blood glucose control of the invention comprises the steps of measuring a plasma glucose (G(t)) signal by means of a continuous glucose monitor (CGM); calculating a glucose level ($\hat{G}(t)$) by using the glucose-insulin model (M), preferably the IVP model, describing the effect of insulin and rescue carbohydrates on glucose; and computing a disturbance term d(t) as:

$$d(t) = \hat{G}(t) - G(t)$$

Independent of the model M selected, an IMC filter Q(s) generates a virtual signal $u_{IMC}(t)$ (in insulin units) that mitigates the effect of d(t) on the output. The term d(t) includes everything not modeled by the model M: external disturbances such as the effect of meal intakes and exercise events, and internal disturbances, such as parametric uncertainty in insulin sensitivity or absorption.

Therefore, reducing the effect of d(t) on the output will also attenuate all these disturbances. The IMC filter, Q(s), is selected as in the two-degree-of-freedom IMC:

$$Q(s) = \frac{u_{IMC}(s)}{d(s)} = F(s) \cdot H^{-1}(s) \tag{2}$$

where s is the Laplace variable. H(s) is the linearization of the model of Eq. 1 (for $u_{resc}(t) = 0$, i.e., the linearized effect of insulin infusion on glucose when $d_1(0) = d_2(0) = 0$) is preferably given by

$$H(s) := \frac{G(s)}{u_T(s)} = \frac{S_I G_0^2}{C_I EGP \left(\frac{1}{p_2}s + 1\right)(\tau_1 s + 1)(\tau_2 s + 1)\left(\frac{G_0}{EGP}s + 1\right)} \tag{3}$$

wherein, if the model M used is the IVP model, $G_0$ is the steady-state glucose value reached for the patient's basal insulin infusion. The filter F(s) is also preferably defined as:

$$F(s) = \frac{k}{(\tau s + 1)^n} \tag{4}$$

where k is the gain of the filter. The order of the filter, n, is set so as to Q(s) is strictly proper transfer function when inverting H(s), that is, the degree of the numerator of Q(s) is lower than the degree of the denominator. The time constant $\tau$ determines the aggressiveness of the filter. Meal intakes and exercise strongly impact plasma glucose in the short term, but they fade by their dynamics.

In addition, due to absorption and measurement lags, the signal d(t) will acknowledge the onset of actual disturbances (e.g., meals, exercise, etc.) with a delay. If $\tau$ is set to a high value, the peak of $u_{IMC}(t)$ will occur much after the disturbance peak; hence reducing the disturbance effect by the filter will be negligible and even counterproductive (e.g., in postprandial control, delayed insulin may lead to hypoglycemia). The filter must quickly react against any deviation in d(t) to reduce the effect of disturbances on glucose. For this reason, $\tau$ is preferably set to two times the sampling time of the CGM reading rate. More preferably, $\tau$ is set to $\tau = 10$ min, since the CGM reading rate is usually 5 min.

The time constant $\tau$ is set to a low value to counteract the insulin absorption delay by infusing a large amount of insulin in a short time. However, this aggressive tuning amplifies measurement noise, leading to an oscillatory signal $u_{IMC}(t)$. The negative values of $u_{IMC}(t)$ would compensate the positive ones given the lowpass-filter nature of the glucose-insulin system that avoids transferring this measurement noise effect to the output.

However, negative values for insulin are not possible since insulin cannot be removed exogenously. If $u_{IMC}(t)$

were delivered without the negative values would cause an insulin over-delivery, lowering the glucose and even leading to hypoglycemia. Thus, the first goal of the switching logic is to ensure that the proposed loop only applies a control action after a disturbance by removing from $u_{IMC}(t)$ the oscillations caused by measurement noise.

The switching logic also adequates the type of control action to the effect of disturbance on the glucose. Insulin is suitable to compensate for the glucose rise following a meal.

However, aerobic low-to-moderate exercise usually leads to a glucose drop and, eventually, hypoglycemia, which is unlikely to be compensated with only an insulin reduction. To compensate for glucose drop, usually related to exercise and insulin overdoses within the postprandial period, the switching logic reduces the tolerated insulin-on-board and suggests rescue carbohydrates to the subject.

Therefore, the second goal of the switching logic is to convert the virtual signal $u_{IMC}(t)$ into three feedforward actions: insulin infusion, rescue carbohydrate suggestion, and insulin-on-board reduction by:

- The insulin $u_{ins}(t)$ is set to 0 if $u_{IMC}(t)$ is lower than a positive threshold $th_{ins}$ to avoid an insulin overdose due to measurement noise amplification in $u_{IMC}(t)$.

- Meal ingestion will likely lead to a glucose rise demanding an $u_{IMC}(t)$ that overpasses $th_{ins}$. To compensate for it, $u_{ins}(t)$ is matched to $u_{IMC}(t)$, to add insulin to the main controller, until it reaches an upper saturation threshold $th_{sat}$, set to avoid overdosing, if $u_{IMC}(t)$ is higher than $th_{ins}$, or substract insulin from the main controller for negative $u_{IMC}(t)$ higher than $th_{resc}$.

- Against a glucose rise, the IMC filter reacts first with a positive peak (above phase), but then, it will have a negative insulin peak. If $u_{IMC}(t)$ is higher than $th_{resc}$, $u_{ins}(t)$ will equate $u_{IMC}(t)$ to subtract insulin from the main controller and, hence avoiding overdosing and the likely related hypoglycemia. If $u_{IMC}(t)$ is lower than $th_{res}$, reducing the insulin from the main controller may be insufficient. Therefore, the negative-valued insulin is converted into rescue carbohydrates suggestions ($u_{resc}(t)$), and $u_{ins}(t)$ zeroed to avoid both types of control actions coupling each other.

The switching logic module converts the "negative insulin" into rescue carbohydrate suggestions to mitigate hypoglycemia.

To this end, first, a virtual unquantized carbohydrate signal, $u_{int}(t)$, is calculated by integrating $u_{IMC}(t)$ in a sliding window of length $t_w$, preferably $t_w = 60$ min, as follows:

$$u_{IMC}^{*}(t) = \begin{cases} u_{IMC}(t) & \text{if} \quad u_{IMC}(t) \le th_{resc} \\ 0 & \text{otherwise} \end{cases} \qquad (5)$$

$$u_{int}(t) = -k_{resc} \int_{t-t_w}^{t} u_{IMC}^{*}(\tau)W(\tau)d\tau - \int_{t-t_w-T_s}^{t-T_s} u_{resc}(\tau)W(\tau)d\tau \qquad (6)$$

where $T_s$ is the sampling time. The first integral in Eq. 6 accumulates the "negative insulin" and transforms it into carbohydrates units (g) through the gain $k_{resc}$. Noise or unimportant glucose drops may lead to small negative values in $u_{IMC}(t)$, i.e., if $-th_{resc} \le u_{IMC}(t) \le 0$. To avoid suggesting rescue carbohydrates when insulin inhibition may suffice, the first integral in Eq. 6 includes the signal $u_{IMC}^{*}$ (t) instead of $u_{IMC}(t)$.

In addition, the forgetting factor $W(t)$ attenuates the earlier values of $u_{IMC}^{*}$ (t) in the sliding window. $W(t)$ is defined by a function $W(t^{*})$ so that:

A) $W(t^{*}) = 1$, for $t^{*}=t$, being t the current time; and
B) $0 \le W(t^{*}) \le 1$, monotone non-decreasing, for $t^{*}$ in $[t-t_w,t]$

wherein monotone non-decreasing means that $W(t_1) \le W(t_2)$ for all $t_1<t_2$, and preferably is given by:

$$W(t^{*}) = e^{-2} \cdot e^{(t^{*}-t+t_w)/30} \qquad (7)$$

for $t^{*} \in [t - t_w, t]$ where t refers to the current time and $t - t_w$ the beginning of the sliding window (when the earliest value of $u_{IMC}^{*}$ (t) is considered), and $t_w=60$ min. The second integral in Eq. 6 subtracts the rescue carbohydrates suggested within the sliding window to avoid increasing $u_{int}(t)$.

The virtual carbohydrate signal, $u_{int}(t)$, must be quantized for user convenience. The quantized rescue signal, $u_{resc}(t)$, follows the next logic:

$$u_{resc}(t) = \begin{cases} \left\lfloor \frac{u_{int}(t)}{\widetilde{cho}} \right\rfloor \cdot \widetilde{cho} \text{ if} & u_{int}(t) \geq 0.5\, \widetilde{cho} \text{ and} \\ & G^*(t) \leq C_2 \text{ and } \Delta t_{resc} > C_1 \\ \widetilde{cho} \qquad \text{if} & G^*(t) \leq C_3 \text{ and} \\ & CGM(t) \leq C_2 \text{ and } \Delta t_{resc} > C_1 \\ 0 & \text{otherwise} \end{cases} \tag{8}$$

where

$$\lfloor \cdot \rfloor$$

denotes the nearest integer operator, $\widetilde{cho}$ is the quantization level, preferably set to 15 g, $\Delta t_{resc}$, the elapsed time between two consecutive rescue carbohydrate suggestions (in min). $C_1$ is a parameter introduced as a delay for avoiding consecutive rescue carbohydrate suggestions, preferably set to 15 min, $C_2$ and $C_3$ are parameters related to mild hypoglycemia (also known as level 1 hypoglycemia) and moderate hypoglycemia (also known as level 2 hypoglycemia) thresholds, preferably set to 70 and 54 respectively, and $G^*(t)$ is a glucose prediction (in mg/dL), which can be computed with the following linear extrapolation:

$$G^*(t) = CGM(t) + C_5 \cdot \frac{dCGM(t)}{dt} \tag{9}$$

wherein $C_5$ is a parameter fixing the time of the glucose prediction, preferably set to 30 min.

According to Eq. 8, the controller suggests rescue carbohydrates in two situations:

- When the system predicts a moderate hypoglycemia risk and the accumulated rescue carbohydrates are large enough. If $u_{int}(t)$ halves the minimum rescue dose the algorithm calculates a rescue carbohydrate suggestion by approximating $u_{int}(t)$ to the nearest multiple of *cho,* If the predicted glucose is outside hypoglycemia risk, the system will not suggest a rescue carbohydrate even though $u_{int}(t) \geq 0.5\, \widetilde{cho}$.
- When the subject is in moderate hypoglycemia, and the glucose tends to a severe hypoglycemia. Here, the system suggests a rescue regardless of the value of $u_{int}(t)$

Preferably *cho* is set to 15 g since available commercial glucose supplements usually contain this amount, e.g., Dex4 (Can-Am Care, Alpharetta, GA, USA), Glutose15 (Paddock Laboratories, Minneapolis, MN, USA), TruePlus (Trividia Health, Fort Lauderdale, FL, USA), etc. Nevertheless, other quantization than 15 g could have been used.

The algorithm considers a minimum elapsed time of $C_3$, preferably set to 15 min, between rescue carbohydrates suggestions to avoid frequent recommendations.

After a rescue carbohydrates suggestion, the switching logic:

- introduces a more strict limitation of the tolerated insulin-on-board of the main controller;
- zeroes $u_{ins}(t)$ during a predefined time $T_1$ following the last rescue carbohydrate suggestion; and
- restores the nominal values of insulin-on-board limitation and $u_{ins}(t)$ when CGM(t) is over a first threshold $Th_1$ and $G^*(t)$ over a second threshold $Th_2$.

Preferably, the predefined time $T_1$ is in the range from 30 to 300 min, and the thresholds $Th_1$ and $Th_2$ are defined as: $CGM(t) \geq 140$ mg/dL and $G^*(t) \geq 180$ mg/dL

The step of introducing a more strict limitation of the tolerated insulin-on-board of the main controller could be done in different ways such as:

- by decreasing an upper limit of insulin-on-board in a given percentage to a percentage between 10% and 90% of its nominal value, preferably 70%,
- by adjusting a gain parameter in the controller so that infused insulin is decreased, or

- by adjusting the cost of a receding horizon based controller so that infused insulin is decreased.

This module preserved the %time in range achieved by the hybrid artificial pancreas, considering carbohydrate counting errors, without a relevant increase in the time in hypoglycemia. The rescues suggested by the controller counteracted the exercise-induced hypoglycemia, allowing more flexibility than insulin-only controllers.

The method of the invention could also comprise an optimization step for tunning the parameters k, $th_{ins}$, $th_{sat}$, $k_{resc}$, $th_{resc}$ by applying a cost defined as:

$$J_{sim} := J_{WAIR} + J_C$$

The term $J_{WAIR}$ penalizes the weighted areas of the CGM exceeding certain thresholds $g_{uu}$, $g_u$, $g_l$ and $g_{ll}$ as follows:

$$J_{WAIR} = a_{uu} \cdot \int_0^{Tsim} (G_{uu}(\tau) - g_{uu}) \, d\tau + a_u \cdot \int_0^{Tsim} (G_u(\tau) - g_u) \, d\tau +$$

$$+ a_l \cdot \int_0^{Tsim} (g_l - G_l(\tau)) \, d\tau + a_{ll} \cdot \int_0^{Tsim} (g_{ll} - G_{ll}(\tau)) \, d\tau +$$

$$+ a_{resc} \cdot \int_0^{Tsim} (G_{resc}(\tau) - g_{resc}) \, d\tau$$

Where Tsim is the simulation length and the parameters $a_{uu}$, $a_u$, $a_l$, $a_{ll}$, and, $a_{resc}$ are non-negative scalars defining the weight of each term. The thresholds are positive scalars defined so that $g_{ll} < g_l < g_u < g_{uu}$.

Signals $G_{uu}(t)$, $G_u(t)$, $G_l(t)$, $G_{ll}(t)$ correspond to the CGM after being saturated to the enclosing thresholds as follows:

$$G_{uu}(t) := \begin{cases} g_{uu} & \text{if} & CGM(t) \le g_{uu} \\ CGM(t) & \text{otherwise} \end{cases}$$

$$G_u(t) := \begin{cases} g_u & \text{if} & CGM(t) \le g_u \\ g_{uu} & \text{if} & CGM(t) > g_{uu} \\ CGM(t) & \text{otherwise} \end{cases}$$

$$G_l(t) := \begin{cases} g_{ll} & \text{if} & CGM(t) < g_{ll} \\ g_l & \text{if} & CGM(t) \ge g_l \\ CGM(t) & \text{otherwise} \end{cases}$$

$$G_{ll}(t) := \begin{cases} g_{ll} & \text{if} & CGM(t) \ge g_{ll} \\ CGM(t) & \text{otherwise} \end{cases}$$

The last addend of $J_{WAIR}$ weights the glucose rebound after rescue carbohydrate suggestion time to better coordinate rescue carbohydrates suggestions and insulin doses.

Signal $G_{resc}(t)$ represents the value of the CGM that overpasses a certain limit of $g_{resc}$ mg/dL in the first $C_6$ min after rescue carbohydrate suggestions. If a meal occurred before the $C_6$ min, $G_{resc}(t)$ was calculated until mealtime as defined in:

$$G_{resc}(t) = \begin{cases} CGM(t) & \text{if} & (CGM(t) \ge g_{resc}) \; and \\ & & t \in [t_{resc}, \min(t_{resc} + C_6, t_{meal})] \\ g_{resc} & \text{otherwise} \end{cases}$$

where $t_{resc}$ and $t_{meal}$ denote the rescue carbohydrates and meals times, respectively.

The second term of the cost function, i.e., $J_C$, constrains the shape and magnitude of the control actions as

defined below:

$$J_C = b_{act} \cdot \max\left(\frac{n_{imc\_act}}{n_{meal}} - 1,0\right) + b_{meal\_resc} \cdot \sum_{i=1}^{n_{meal\_resc}} meal\_resc_i + $$

$$+ b_{ex\_resc} \cdot \max\left(\frac{\sum_{i=1}^{n_{ex\_resc}} ex\_resc_i}{C_7 n_{ex\_sessions}} - 1,0\right)$$

where:

- The first addend penalizes situations wherein the number of IMC activations in insulin mode (n_imc_act) is larger than the number of meals (n_meal) in the optimization scenario. The parameter $b_{act}$ is the weight of the addend and could be any non-negative scalar.
- The second addend constrains the total amount of rescues given after meals. The carbohydrate amount given at meal i is defined as meal_resc_i and $n_{meal\_resc}$ denotes the number of rescues given after meals. The parameter $b_{meal\_resc}$ is the weight of the addend and could be any non-negative scalar.
- The third addend penalizes situations wherein the amount of suggested carbohydrate per exercise event exceeds a certain value of $C_7$ g. The carbohydrate amount suggested after exercise i is denoted as ex_resc_i, and n_ex_resc and n_ex_sessions define the number of exercise-related rescues and exercise events, respectively. The parameter $b_{ex\_resc}$ is the weight of the addend and could be any non-negative scalar.

Preferably, the thresholds $g_{resc}$, $g_{ll}$, $g_l$, $g_u$ and $g_{uu}$ are in the range from 40 to 400 mg/dL; $C_7$ in the calculation of $G_{resc}(t)$ is in the range from 5 to 300 min; and the tolerable amount of suggested carbohydrate per exercise event in $J_C$, represented by $C_7$, is between 5 g and 100 g.

The invention also refers to a computer program adapted for carrying out the steps of the method of the invention defined by using the calculation unit of the add-on module of the invention. The invention also refers to a computer readable storage medium comprising the computer program.

## DESCRIPTION OF THE DRAWINGS

To complement the description being made and in order to aid towards a better understanding of the characteristics of the invention, in accordance with a preferred example of practical embodiment thereof, a set of drawings is attached as an integral part of said description wherein, with illustrative and non-limiting character, the following has been represented:

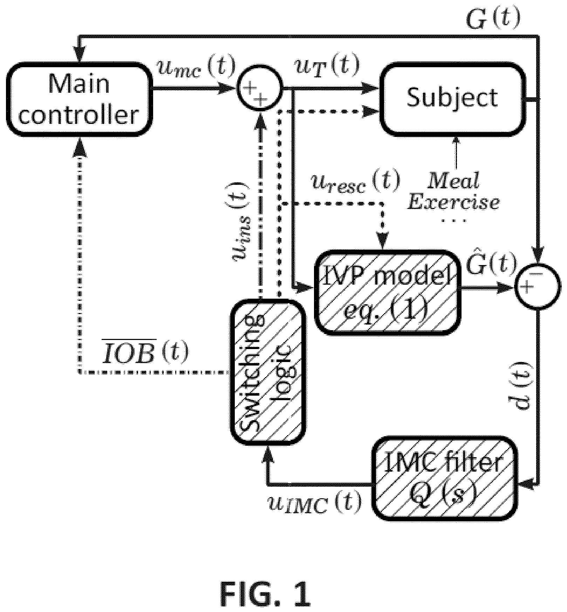

Figure 1.- shows an overview of the control method of the invention, wherein blocks with stripped pattern represent the proposed add-on module: an Internal Model Control (IMC) loop (IVP, Identifiable Virtual Patient model, and IMC filter) with a non-linear logic (Switching logic). The add-on module provides three actions: insulin (dashed and double dotted line, $u_{ins}(t)$), rescue carbohydrates (dotted line, $u_{resc}(t)$), and maximum insulin on board (IOB) limit command (dashed and dotted line, $\overline{IOB}(t)$).

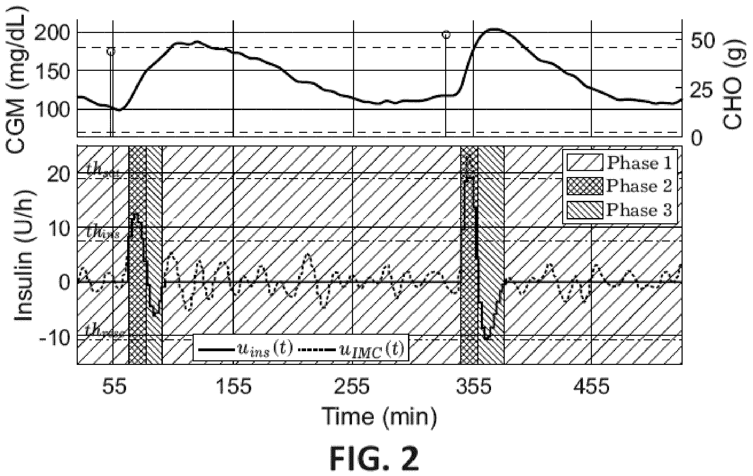

Figure 2.- shows the results of a control logic to compensate meals. The switching logic processes the virtual action $u_{IMC}(t)$ in three phases: dead-zone (Phase 1), glucose rise mitigation (Phase 2), and later hypoglycemia prevention (Phase 3). It results in the insulin infusion $u_{ins}(t)$ added to the main controller. Parameters $th_{ins}$, $th_{sat}$, and $th_{resc}$ are, respectively, the thresholds to inhibit $u_{IMC}(t)$, saturate it, or convert it into rescue carbohydrates suggestions, respectively.

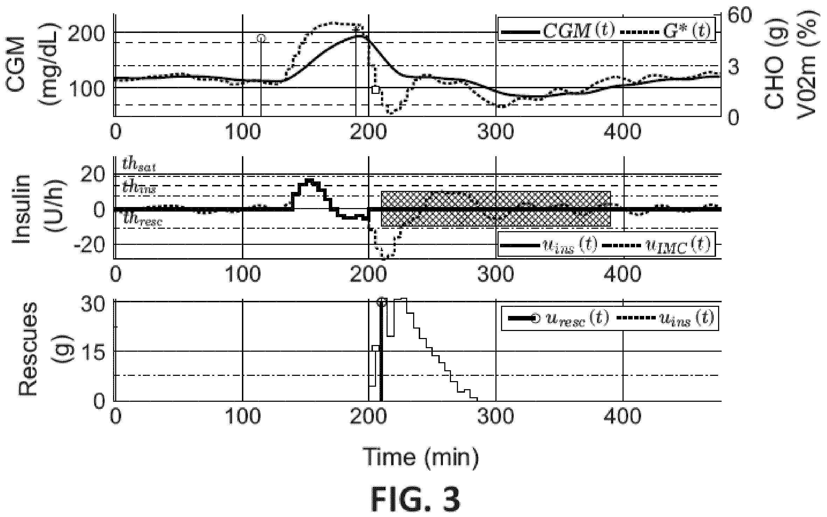

Figure 3.- shows the results of a control logic to compensate exercise. The switching logic converts the negative insulin of the IMC ($u_{IMC}(t)$, middle panel) into a continuous carbohydrate signal ($u_{int}(t)$, bottom panel). If the predicted glucose ($G^*(t)$, upper panel) is in hypoglycemia and $u_{int}(t) \geq 7.5$ (dashed and dotted line in bottom panel), the algorithm suggests a rescue $u_{resc}(t)$ (bottom panel) by quantizing $u_{int}(t)$. Hollow squares illustrate that rescue carbohydrates are inhibited if no hypoglycemia risk exists. Insulin $u_{ins}(t)$ is inhibited after the rescue carbohydrate suggestion (stripped pattern area in middle panel)

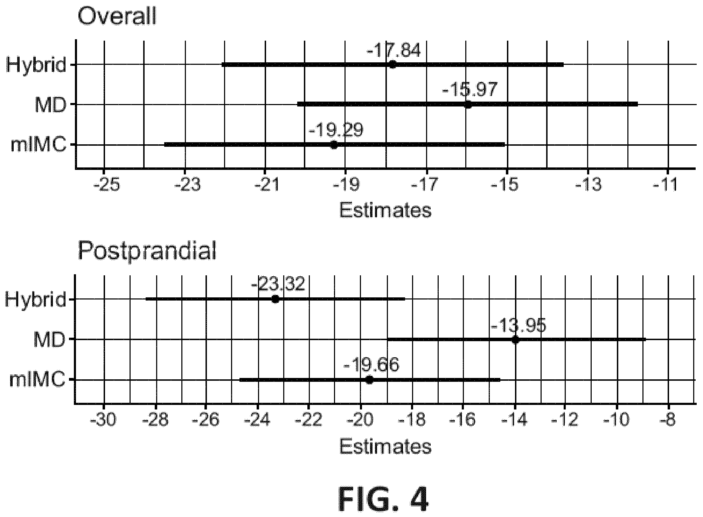

Figure 4.- shows a mean difference of the percentage time above 180 mg/dL regarding the controller without meal compensation (NoComp). The labels indicate the mean difference between every controller with meal compensation (Hybrid, MD, mIMC) and NoComp obtained using robust random-intercept models. Hybrid represents a controller with meal announcement. MD represents a controller without meal announcement and postprandial hyperglycemia compensation through a meal detection scheme. mIMC represents the method of the invention. Lines represent the Wald 95%-interval confidence. The upper panel refers to the percentage time of CGM above 180 mg/dL within 30 days of simulation, while only the 3 h after each meal are considered in the bottom panel.

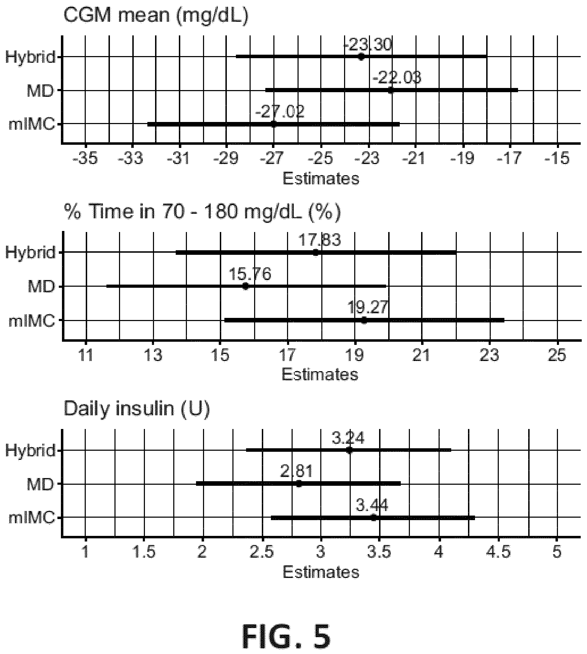

Figure 5.- shows a mean difference of CGM mean, time in range, and daily insulin regarding the controller without meal compensation (NoComp). The labels indicate the mean difference between every controller with meal compensation (Hybrid, MD, mIMC) and NoComp obtained using robust random-intercept models. Hybrid represents a controller with meal announcement. MD represents a controller without meal announcement and postprandial hyperglycemia compensation through a meal detection scheme. mIMC represents the method of the invention. Lines represent the Wald 95%-interval confidence.

The upper panel refers to the CGM mean, the middle panel to the percent time in 70 -180 mg/dL, and the bottom panel to the daily insulin. All metrics correspond to 30 days of simulation.

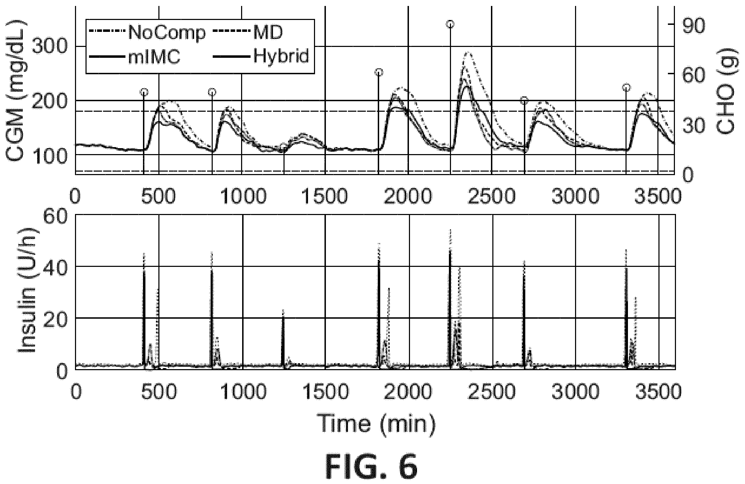

Figure 6.- shows a graph of populational glucose and insulin profiles. It shows 2 of the 30 days of the simulation comparing four meal compensation techniques: absence of meal compensation (NoComp), announced-based compensation (Hybrid), meal-detector-based compensation (MD), and proposed approach (mIMC). The lines represent the median of the 10 virtual adults. The hollow circles in the upper panel represent meal events whose carbohydrate contents are shown on the right axis.

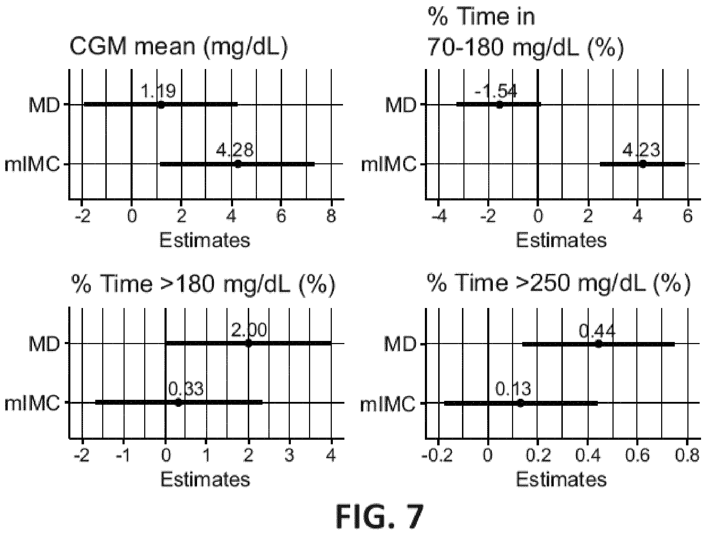

Figure 7.- shows a mean difference of CGM mean, time in range, and time in hyperglycemia regarding the controller without exercise compensation (NoExComp), that is, the mIMC controller with the rescue carbohydrate suggestion feature deactivated. The labels indicate the estimated mean difference of the controllers MD and mIMC regarding the controller NoExComp obtained using robust random-intercept models. Lines represent the Wald 95%-interval confidence. All metrics correspond to 30 days of simulation.

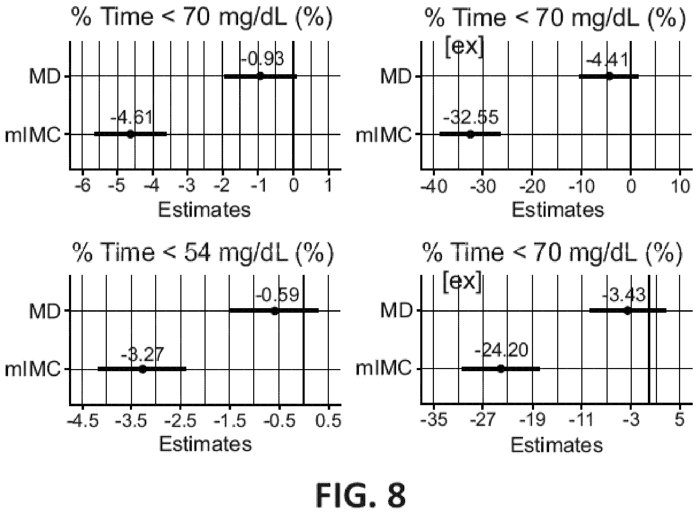

Figure 8.- shows a mean difference of time in hypoglycemia regarding the controller without exercise compensation (NoExComp). The labels indicate the estimated mean difference between the controllers MD and mIMC, and the controller NoExComp using robust random-intercept models. Lines represent the Wald 95%-interval confidence. The term "ex" added to the name of the metrics denote that the metric corresponded to the first 3 h after each exercise event, while the remaining metrics considered the 30 days of simulation.

## PREFERRED EMBODIMENT OF THE INVENTION

Figure 1 illustrates a module (see blocks with stripped pattern) that replaces those announcements with only minor modifications in the main controller once plugged into it.

The parameters of the model (Eq. 1) were identified from the 10 virtual adults of the academic version of the UVa/Padova simulator since the controller was evaluated with this cohort. Table 1 includes the identified parameters.

| Subject | $EGP$ (mg/dL/min) | $SI$ (mL/$\mu$U/min) | $Vg$ (dL) | $p_2$ (1/min) |
|---|---|---|---|---|
| 1 | 1.32 | $5.17 \cdot 10^{-4}$ | $2.35 \cdot 10^2$ | $2.53 \cdot 10^{-3}$ |
| 2 | 1.20 | $4.24 \cdot 10^{-4}$ | $2.48 \cdot 10^2$ | $4.08 \cdot 10^{-2}$ |
| 3 | 1.05 | $3.35 \cdot 10^{-4}$ | $1.85 \cdot 10^2$ | $2.03 \cdot 10^{-3}$ |

| | | | | |
|---|---|---|---|---|
| 4 | 1.49 | $7.23 \cdot 10^{-4}$ | $2.9 \cdot 10^{2}$ | $3.39 \cdot 10^{-3}$ |
| 5 | 0.762 | $2.52 \cdot 10^{-4}$ | $5.81 \cdot 10^{2}$ | $1.12 \cdot 10^{-2}$ |
| 6 | 0.925 | $2.43 \cdot 10^{-4}$ | $1.83 \cdot 10^{2}$ | $4.08 \cdot 10^{-2}$ |
| 7 | 0.916 | $2.84 \cdot 10^{-4}$ | $2.54 \cdot 10^{2}$ | $2.03 \cdot 10^{-3}$ |
| 8 | 0.925 | $2.39 \cdot 10^{-4}$ | $4.57 \cdot 10^{2}$ | $6.9 \cdot 10^{-3}$ |
| 9 | 0.699 | $3.26 \cdot 10^{-4}$ | $2.77 \cdot 10^{2}$ | $4.08 \cdot 10^{-2}$ |
| 10 | 1.53 | $6.03 \cdot 10^{-4}$ | $2.99 \cdot 10^{2}$ | $6.01 \cdot 10^{-3}$ |

| Populational | $A_g^{resc}$ (unitless) | $CI$ (mL/min) | $GEZI$ (1/min) | $\tau_1$ (min) |
|---|---|---|---|---|
| | 0.900 | $1.22 \cdot 10^{3}$ | $2.35 \cdot 10^{-3}$ | 74.3 |
| | $\tau_2$ (min) | $\tau_{resc}$ (min) | $A_g^{meal}$ (unitless) | $\tau_{meal}$ (min) |

Table 1. Control model parameters corresponding to the virtual adults in UVa/Padova simulator. The first column represents the subject identifier in the simulator. Parameters *EGP*, *SI*, *V g*, and $p_2$ resulted from optimization. Parameters *CI*, *GEZI*, $\tau_1$, and $\tau_2$ are populational values. Parameters $A_g^{resc}$ and $\tau_{resc}$ were chosen to represent a fast-acting carbohydrate rescue; they are populational values too. Meal model parameters ($A_g^{meal}$ and $\tau_{meal}$).

The synthetic dataset for identification corresponds to a 2-week basal-bolus therapy of 3 daily meals. Populational values were considered for the absorption dynamics of rescue carbohydrates, and thus parameters in equations Eq.1d-Eq.1e were excluded from the identification process.

For the identification of the rest of the model parameters, an additional meal model was considered in order to match clinical data (which include meals).

However, it must be remarked that this meal model was not part of the IMC controller, since meals are unannounced and thus, they are treated as output disturbances. The meal absorption model matches the structure of (1d)-(1e) but with different signals and parameters involved, e.g., larger doses, longer time constants, etc. The identification process used information available in practical settings (CGM reading, insulin infusion, meal dose, and mealtime). Identifiability issues - such as parameter correlation - were handled by selecting the parameters according to the structural identifiability, the global sensitivity, and the collinearity index.

Therefore, the method of the invention relates to a control method having five parameters requiring an individual tuning for the 10 virtual adults in the simulator: the gain of F(s) ($k_{ins}$), the gain factor converting insulin into carbohydrates ($k_{resc}$), and the three thresholds of the switching logic ($th_{ins}$, $th_{sat}$, and $th_{resc}$). These parameters have been adjusted through the optimization procedure described below.

For each subject, the worst-case within 12 simulations of the same virtual adult, with different instances of parametric variability, is minimized. Each simulation consisted of a 7-day ($T_{sim}$ = 10080 min) scenario with 3 daily meals and 1 daily exercise session.

The cost applied to each simulation is defined as:

$$J_{sim} := J_{WAIR} + J_C \qquad (10)$$

This cost penalizes the weighted areas in risk ($J_{WAIR}$) and constrains the magnitude or shape of the control actions ($J_C$). The weighted areas in risk consider the areas of the CGM exceeding the thresholds 54, 70, 180, and 250 as follows:

$$
\begin{aligned}
J_{WAIR} \quad &= a_{uu} \cdot \int_0^{Tsim} (G_{uu}(\tau) - 250)\, d\tau + a_u \cdot \int_0^{Tsim} (G_u(\tau) - 180)\, d\tau + \\
&+ a_l \cdot \int_0^{Tsim} (70 - G_l(\tau))\, d\tau + a_{ll} \cdot \int_0^{Tsim} (54 - G_{ll}(\tau))\, d\tau + \\
&+ a_{resc} \cdot \int_0^{Tsim} (G_{resc}(\tau) - 140)\, d\tau
\end{aligned}
\qquad (11)
$$

where the scalars $a_{uu} = 175$, $a_u = 1$, $a_l = 5000$, $a_{ll} = 10000$, $a_{resc} = 50$ are the weights. The weights above were tuned so that the areas in hypoglycemia cost more than those in hyperglycemia.

This flexibility in configuring the optimal performance is not possible in other approaches that rely on standard metrics to define the cost. All the integrals were calculated using the trapezoidal rule. Signals $G_{uu}(t)$, $G_u(t)$, $G_l(t)$, $G_{ll}(t)$ in Eq. 11 correspond to the CGM after being saturated to the enclosing thresholds as follows:

$$
G_{uu}(t) := \begin{cases} 250 & \text{if} & CGM(t) \leq 250 \\ CGM(t) & \text{otherwise} \end{cases}
\qquad (12)
$$

$$
G_u(t) := \begin{cases} 180 & \text{if} & CGM(t) \leq 180 \\ 250 & \text{if} & CGM(t) > 250 \\ CGM(t) & \text{otherwise} \end{cases}
\qquad (13)
$$

$$
G_l(t) := \begin{cases} 54 & \text{if} & CGM(t) < 54 \\ 70 & \text{if} & CGM(t) \geq 70 \\ CGM(t) & \text{otherwise} \end{cases}
\qquad (14)
$$

$$
G_{ll}(t) := \begin{cases} 54 & \text{if} & CGM(t) \geq 54 \\ CGM(t) & \text{otherwise} \end{cases}
\qquad (15)
$$

An insulin overdose might cause a glucose drop that the controller would compensate with rescue carbohydrates suggestions. The last addend of Eq. 11 weights the glucose rebound after rescue carbohydrate suggestion time to better coordinate rescue carbohydrates suggestions and insulin doses.

Signal $G_{resc}(t)$ represents the value of the CGM that overpasses 140 mg/dL in the first 3 h after rescue carbohydrate suggestions. If a meal occurred before the 3h, $G_{resc}(t)$ was calculated until mealtime as defined in:

$$
G_{resc}(t) = \begin{cases} CGM(t) & \text{if} & \begin{aligned} &(CGM(t) \geq 140)\ and \\ &t \in [t_{resc}, \min(t_{resc} + 3h, t_{meal})] \end{aligned} \\ 140 & \text{otherwise} \end{cases}
\qquad (16)
$$

where $t_{resc}$ and $t_{meal}$ denote the rescue carbohydrates and meals times, respectively.

Mealtimes were available to define the cost function but were unknown to the controller.

The cost $J_C$ penalizes the number of times the IMC activates the insulin mode (Phase 2 of Figure 2) for $u_{ins}(t)$ to behave like a bolus: being active a short time with large insulin doses rather than being continuously activated with reduced doses. The cost $J_C$ also constrains the size of rescue carbohydrates. To reduce the risk of compensating insulin overdosing with rescue carbohydrates, the carbohydrate suggestions followed by meals (meal rescue carbohydrates) were weighted more than those followed by exercise sessions (exercise rescue carbohydrates). For the exercise-related rescue carbohydrates, the average rescue size per exercise event was limited to 45 g. The expression of $J_C$ is the following:

$$J_C = b_{act} \cdot \max\left(\frac{n_{imc\_act}}{n_{meal}} - 1,0\right) + b_{meal\_resc} \cdot \sum_{i=1}^{n_{meal\_resc}} meal\_resc_i +$$

$$+ b_{ex\_resc} \cdot \max\left(\frac{\sum_{i=1}^{n_{ex\_resc}} ex\_resc_i}{45 n_{ex\_sessions}} - 1,0\right) \tag{17}$$

where $b_{act}$ = 1400, $b_{meal\_resc}$ = 15000, and $b_{ex\_resc}$ = 4500 are weights. Terms $n_{imc\_act}$, $n_{meal\_resc}$, $n_{ex\_resc}$, $n_{ex\_session}$ denote the number of times the IMC enters Phase 2, the number of meal-related rescue carbohydrates, the number of exercise-related rescue carbohydrates, and the number of exercise sessions, respectively. $meal\_resc_i$ represents the meal rescue sizes (from i = 1 to i = $n_{meal\_resc}$) and $meal\_ex_i$ the exercise rescue sizes (from i = 1 to i = $n_{ex\_resc}$).

The min-max problem was solved with the Covariance Matrix - Adaptation Evolution Strategy (CMA-ES) algorithm, a black-box search optimizer suitable for non-linear or non-convex problems. Table 2 includes the starting values and the bounds of the parameters.

Table 2.

|  | Initial value | Lower limit | Upper limit |
|---|---|---|---|
| $k_{ins}$ (-) | 0.5 | 0.01 | 1 |
| $th_{ins}$ (U/h) | 5 | 1 | 30 |
| $th_{sat}$ (U/h) | 10 | 1 | 30 |
| $th_{resc}$ (U/h) | 1 | 0.05 | 5 |
| $k_{resc}$ (g/U/h) | 0.1 | 0.0005 | 0.5 |

The above method of tuning the controllers is feasible for in-silico studies. To provide a starting tuning for a clinical trial, the optimal parameters were related by means of a regression to standard parameters of the open-loop therapy: the weight (BW, in kg), the total daily insulin (TDI, in U), the basal insulin ($u_b$, in U/h), the carbohydrate-to-insulin ratio (CR, in g/U), and the correction factor (CF, in mg/dL/U). For each optimal parameter, a relation to open-loop parameters was found as follows:

1. The 80 linear models that fit the corresponding optimal parameter with the lowest root sum of squares were selected. Models had up to 8 coefficients, including pairwise interactions of the open-loop parameters. The selection was performed with the function regsubset of the R software.
2. To mitigate the risk of overfitting, the selected models were fitted using leave-one-out cross-validation.
3. The final model was the model with the lowest number of coefficients that had an accurate root-mean-squared error of the cross-validation and satisfied the diagnosis assumptions (normality and homoscedasticity of the residuals).

The proposed add-on module was implemented in the UVA/Padova simulator for validation purposes. The simulator emulates the 5-min sampling time of the CGM; hence the add-on module must be implemented in discrete time.

For the implementation, the model in Eq. 1 was discretized with Euler approximation using a sampling period of 5 min ($T_s$ = 5 min). The filter Q(s) in Eq. 2 and the integral in Eq. 6 were discretized using a Tustin approximation, also with $T_s$ = 5 min.

The validation targets three purposes:

1) to determine whether the regression-based tuning maintains the performance of the optimal tuning,
2) to assess the controller against meals, and
3) to assess the controller against meals and exercise.

The fit of the regression model to the corresponding optimal parameter was assessed with the coefficient of determination $R^2$ and the root-mean-squared error of the cross-validation ($RMSE_{loocv}$).

To study if the regression-based tuning degraded the performance of the optimal tuning, glucose percentage time-related metrics were compared (the %time in range, the %time in hyperglycemia, and the %time in hypoglycemia).

To this end, both tunings were simulated for 10 virtual adults using the UVa/Padova simulator.

The simulation consisted of a 30-day scenario including 3 daily meals, with random sizes and timing, and 1 daily exercise session. Defining the daily meals by:

- For breakfast: a median of 49.5 g, with an interquartile range of 33.0 g, 55.0 g at a median of 6.92 h and at an interquartile range of 6.75 h, 7.08 h.
- For lunch: a median of 81.0 g, with an interquartile range of 72.0 g, 93.0 g at a median of 13.75 h and at an interquartile range of 13.58 h, 14.17 h.
- For dinner: a median of 64 g, with an interquartile range of 54 g, 79 g at a median of 20.9 h and at an interquartile range of 20.8 h, 21.1 h.

Exercise effect on glucose was simulated through a variation of insulin sensitivity. This exercise model corresponds to an aerobic exercise of 60 min at 50% of $VO_{2max}$, approximately. The exercise time was set up to 240 min after one meal of the day - 12 exercise events after breakfast, 11 after lunch, and 7 after dinner - following a uniform distribution. Exercise events beyond midnight were avoided.

The simulation also included multiple sources of variability: CGM noise, one-day period sinusoidal-type insulin sensitivity variation with random amplitude and phase, variation of subcutaneous insulin absorption rate at each meal following a uniform distribution of $\pm 30\%$, and variability of the meal absorption parameters.

The goal of the validation performance against meals is to quantize the improvement regarding the main controller without any meal compensation (henceforth denoted as NoComp) of three controllers with meal compensation: 1) the main controller with the IMC loop, tuned with the regression model (denoted as mIMC), 2) the main controller with the meal-announcement free compensation feature, based on a super-twisting meal detector (referred as MD), and 3) the main controller with meal announcements but considering errors in the estimation of the carbohydrates (denoted as Hybrid).

The simulation features - duration, number of subjects, variability, meal size, and timing-were identical to the ones described before, but without considering exercise.

To assess the controllers, apart from the standard metrics, percentage-time-related metrics within the postprandial period (from the mealtime until 3 h after each meal) were calculated. Since the mIMC might compensate insulin over-delivery with rescue carbohydrates suggestions, the percentage of meals requiring at least one rescue and the mean size of the rescue carbohydrates suggested for those meals were also reported.

A validation performance against exercise is also carried out, being assessed the likely benefits of the rescue carbohydrate suggestion feature of the mIMC to counteract exercise-induced hypoglycemia.

To this end, the proposed controller mIMC was compared to two insulin-only controllers: 1) the mIMC controller with the rescue carbohydrate suggestion feature deactivated (denoted as NoEx-Comp), and 2) the meal-detector based controller, i.e., MD.

The simulation scenario was identical to the one described before, with exercise events. Apart from the standard metrics, the following exercise-related metrics were computed: the %time in hypoglycemia within the exercise period (from the exercise time to 3 h after it), the %time above 140 mg/dL up to 3 h after each rescue, the percentage of exercise events needing at least one rescue carbohydrate, and the mean rescue size suggested for those events.

In the simulations, subjects ingested the suggested carbohydrates in the precise time and size.

Results were analyzed with a regression-based inference approach and Wald 95% confidence intervals. Since all the simulations in the study shared the virtual cohort, the independence condition assumed by linear models fails.

Mixed-effect models can handle the dependency of the virtual subjects in the sample. For each of the analyzed metrics, the following random-intercept mixed-effect model, individualized for each subject, was fitted:

$$y_{sub} = \beta_0 + S_{sub} + \sum_{i=1}^{n_C-1} \beta_i x_i + e_{sub} \tag{18}$$

where $y_{sub}$ is the corresponding metric value for a given subject, sub, and $n_C$ is the number of controllers to be compared. $S_{sub}$ is the random intercept, and $e_{sub}$ are the residuals, following both a zero-mean normal distribution. Fixed coefficient $\beta_0$ is the intercept, and coefficients $\beta_i$ can be interpreted as the mean difference regarding the intercept since $x_i$ is a dichotomous dummy variable related to the controller to be compared with the intercept. These coefficients inform about the effect size of the differences between structures, a more valuable information than the significance analysis of P-values, especially for in silico analysis where P-values are controversial.

The mixed-effect model was fitted with a robust method of the R software to handle the outliers appearing in the data.

Table 3 includes the optimal parameters of the controller described before. The related regression equations (Table 4) accurately fit the optimal parameters.

Table 3. The first column represents the virtual adult identifier in the UVa/Padova Simulator.

| Subject | $k_{ins}$ (-) | $th_{min}$ (U/h) | $th_{min}$ (U/h) | $k_{resc}$ (g/U/h) | $th_{resc}$ (U/h) |
|---|---|---|---|---|---|
| 1 | 0.04 | 4.43 | 9.38 | 0.09 | 0.48 |
| 2 | 0.17 | 1.46 | 14.34 | 0.31 | 0.15 |
| 3 | 0.03 | 1.08 | 13.20 | 0.06 | 0.53 |
| 4 | 0.08 | 5.50 | 12.19 | 0.12 | 0.07 |
| 5 | 0.11 | 3.72 | 25.95 | 0.09 | 0.07 |
| 6 | 0.25 | 7.57 | 19.62 | 0.12 | 2.08 |
| 7 | 0.02 | 5.42 | 10.25 | 0.09 | 0.12 |
| 8 | 0.14 | 6.83 | 15.22 | 0.05 | 2.45 |
| 9 | 0.34 | 2.34 | 8.08 | 0.16 | 0.72 |
| 10 | 0.14 | 9.69 | 15.69 | 0.09 | 0.35 |

The second, third, and fourth columns include the parameters used for meal compensation, while the remaining columns correspond to the exercise compensation.

Table 4. Regression equations of the controller's parameters and related goodness of t metrics, which are the adjusted coefficient of determination (adjR2) for multivariable regression models and root-mean-squared error of the leave-one out cross-validation (RMSEloocv). The five models have a low RMSEloocv and acceptable coefficients of determination.

| Subject | adjR$^2$ | RMSE$_{loocv}$ |
|---|---|---|
| $\hat{k} = 14.2 + 6.17 \cdot 10^{-2} \cdot TDI - 2.59 \cdot u_b - -1.61 \cdot 10^{-3} \cdot BW \cdot CF + 3.93 \cdot 10^{-3} \cdot BW \cdot CR - -4.67 \cdot 10^{-3} \cdot CF \cdot TDI - 6.57 \cdot 10^{-3} \cdot CR \cdot TDI$ | 0.972 | 0.0471 |
| $\widehat{th}_{ins} = -51.6 + 28.9 \cdot CR + 0.872 \cdot BW \cdot u_b - -2.53 \cdot 10^{-2} \cdot BW \cdot CF - 12.9 \cdot CR \cdot u_b - -0.226 \cdot CF \cdot CR$ | 0.752 | 1.95 |
| $\widehat{th}_{sat} = 4.01 \cdot 10^2 - 1.05 \cdot 10^2 \cdot u_b - 20.8 \cdot CR - -8.82 \cdot 10^{-2} \cdot BW \cdot CF + 0.209 \cdot BW \cdot CR + +0.150 \cdot CF \cdot CR$ | 0.890 | 3.16 |
| $\hat{k}_{resc} = -3.02 - 7.6 \cdot 10^{-2} \cdot CR + 3.11 \cdot 10^{-4} \cdot BW \cdot TDI - -1.74 \cdot 10^{-2} \cdot BW \cdot u_b + 1.76 \cdot 10^{-4} \cdot BW \cdot CF + +8.18 \cdot 10^{-2} \cdot CF \cdot u_b$ | 0.776 | 0.0676 |
| $\widehat{th}_{resc} = -12.3 - 0.133 \cdot BW - 0.295 \cdot TDI +$ | 0.955 | 0.209 |

Furthermore, the mean difference in the metrics %time in 70-180 mg/dL (0.303%, CI:[-1.30, 1.91]), %time above 180 mg/dL (-0.470%, CI:[-2.29, 1.35]), and %time below 70 mg/dL (0.0576%, CI:[-0.199, 0.315]) are negligible, which indicate that the regression-based tuning preserves the performance achieved by the optimal tuning.
The controllers featuring meal compensation (Hybrid, MD, or mIMC) outperform NoComp, as shown in Table 5.

Table 5. Performance metrics of meal compensation. Four meal compensation techniques, which share the same main controllers, were compared: absence of meal compensation (NoComp), announced-based compensation (Hybrid), meal-detector-based compensation (MD), and proposed approach (mIMC). Metrics are expressed in median [25th percentile, 75th percentile] of the 10 virtual adults. Overall metrics aggregate the entire simulation period (30 days), while Postprandial control metrics refer to a specific period of the postprandial: percent of time-related metrics aggregate the 3-h period after the meal, and rescue-related metrics aggregate the meal-to-meal period.

| | NoComp | Hybrid | MD | mIMC |
|---|---|---|---|---|
| **Overall** | | | | |
| Mean CGM (mg/dL) | 161.6 [158.9, 189.5] | 140.6 [139.0, 154.2] | 141.8 [139.0, 145.6] | 140.0 [132.9, 144.8] |
| CV (%) % of time CGM | 30.5 [28.7, 33.7] | 25.2 [23.4, 26.5] | 25.8 [24.4, 29.2] | 25.2 [24.4, 28.6] |
| > 250 mg/dL (%) | 8.2 [4.8, 19.5] | 1.2 [0.3, 2.3] | 1.9 [0.7, 4.0] | 1.4 [0.3, 2.3] |
| > 180 mg/dL (%) | 31.8 [29.9, 45.2] | 16.0 [13.6, 24.1] | 17.3 [16.2, 20.4] | 14.7 [11.8, 22.1] |
| 70-180 mg/dL (%) | 68.2 [54.8, 70.1] | 84.0 [75.9, 86.4] | 81.9 [79.3, 83.8] | 85.1 [77.9, 88.1] |
| < 70 mg/dL (%) | 0.0 [0.0, 0.0] | 0.0 [0.0, 0.0] | 0.0 [0.0, 0.1] | 0.0 [0.0, 0.1] |
| < 54 mg/dL (%) | 0.0 [0.0, 0.0] | 0.0 [0.0, 0.0] | 0.0 [0.0, 0.0] | 0.0 [0.0, 0.0] |
| Daily insulin (U) | 34.7 [29.8, 37.4] | 38.6 [34.0, 41.3] | 38.3 [32.9, 39.9] | 38.5 [34.1, 41.4] |
| **Postprandial control** | | | | |
| % of time CGM | | | | |
| > 250 mg/dL (%) | 17.5 [9.4, 28.3] | 2.6 [0.9, 4.2] | 5.1 [1.7, 9.4] | 3.5 [0.7, 5.9] |
| > 180 mg/dL (%) | 57.2 [49.1, 62.3] | 34.0 [25.7, 35.5] | 41.4 [37.6, 47.5] | 34.9 [29.4, 45.5] |
| < 70 mg/dL (%) | 0.0 [0.0, 0.0] | 0.0 [0.0, 0.0] | 0.0 [0.0, 0.0] | 0.0 [0.0, 0.0] |
| < 54 mg/dL (%) | 0.0 [0.0, 0.0] | 0.0 [0.0, 0.0] | 0.0 [0.0, 0.0] | 0.0 [0.0, 0.0] |
| Meals needing rescues(%) | - | - | - | 0.0 [0.0, 1.1] |
| Mean rescues (g) | - | - | - | 15.0 [15.0, 15.0] |

The improvement is statistically significant since all the fixed-effect coefficients of Figure 4 and Figure 5 -interpreted as the mean difference of each controller regarding NoComp - are far from zero.

Since the confidence intervals in Figure 4 and Figure 5 overlap among controllers, all the controllers with meal compensation improve to a similar degree the performance of NoComp, with the advantage that the MD and mIMC controllers free subjects from meal announcements.

Figure 6 shows the glucose and insulin traces of 2 of the 30 days of the simulation. Some behavioral differences exist between the controllers. The meal announcement in the hybrid controller improves the early phase of the postprandial with a lower time in hyperglycemia, as seen in the bottom panel of Figure 4, and a lower postprandial peak, as seen in Figure 6.

The mIMC tends to be more aggressive, allowing a more rapid recovery than the hybrid controller after large meal, as shown in fifth meal in Figure 6. The price to pay to achieve a similar time in range than the hybrid controller, but without announcement, is a steeper glucose drop after the postprandial (Figure 6).

In no subject, the glucose drops below 54 mg/dL and the %time below 70 mg/dL never overpasses the 0.5%, which is far from the accepted 4% threshold indicated in the literature.

Although the rescue suggestion feature plays a role in avoiding hypoglycemia events, its use was sparse: for most of the subjects, the controller did not suggest any rescue; for two of them, the controller only recommended 15 g; and only for the two remaining ones, the controller recommended more than one rescue, 10 and 12 rescues, always of 15 g.

Finally, the MD achieves a slightly longer time in hyperglycemia than the mIMC, as shown in Table 5. Since the MD does not suggest rescues, it cannot mitigate the glucose drop. As a result, unlike the mIMC, one virtual subject had severe hypoglycemia.

The results on the performance of exercise control are also shown. The carbohydrate suggestion feature in the mIMC significantly reduces the time in moderate and severe hypoglycemia, as seen in Figure 8, compared to when the rescue module is unable (NoExComp).

The meal-detector-based controller (MD) performed like NoExComp as concluded from the confidence intervals, they are small and include the 0, as can be seen in Table 6 and Figure 8). Therefore, the flexibility of insulin-only controllers against exercise-induced hypoglycemia is limited.

The mIMC suggested a median of 27.2 g per exercise session to handle the exercise-induced glucose drop, as shown in Table 6. This value is coherent with other results of unannounced exercise events in the literature.

Table 6. Performance against exercise. It includes the results of three controllers: the meal-detector-based controller (MD), the proposed controller (mIMC), and the proposed controller disabling the rescue suggestion module (NoExComp). Metrics are expressed in median [25th percentile, 75th percentile] of the 10 virtual adults. Overall metrics aggregate the entire simulation period (30 days), while Exercise control metrics refer to a specific period after the exercise: percent of time-related metrics aggregate the 3-h period after the exercise, and rescue-related metrics aggregate the exercise-to-exercise period.

| | | | NoExComp | MD | mIMC |
|---|---|---|---|---|---|
| **Overall** | | | | | |
| | Mean CGM (mg/dL) | | 138.2 [132.1, 141.7] | 136.9 [135.6, 138.5] | 141.9 [136.5, 146.2 |
| | CV (%) | | 33.3 [31.4, 34.4] | 34.1 [33.1, 35.1] | 29.6 [27.4, 32.0] |
| | % of time CGM | | | | |
| | | > 250 mg/dL (%) | 1.6 [0.8, 2.9] | 2.2 [1.5, 3.6] | 1.6 [1.0, 2.9] |
| | | > 180 mg/dL (%) | 19.2 [13.5, 21.1] | 19.2 [18.1, 20.6] | 19.8 [13.6, 20.8] |
| | | 70-180 mg/dL (%) | 75.5 [72.7, 81.5] | 76.5 [71.6, 77.0] | 79.6 [77.5, 85.5] |
| | | < 70 mg/dL (%) | 5.3 [4.5, 6.6] | 4.6 [4.0, 5.1] | 0.9 [0.4, 1.1] |
| | | < 54 mg/dL (%) | 3.7 [2.2, 4.1] | 2.8 [2.0, 3.4] | 0.0 [0.0, 0.0] |
| | Daily insulin (U) | | 37.9 [33.0, 40.7] | 37.6 [31.8, 39.6] | 37.7 [33.2, 40.6] |
| | Daily CHO (g) | | 0.0 [0.0, 0.0] | 0.0 [0.0, 0.0] | 27.5 [23.9, 31.0] |
| **Exercise control** | | | | | |
| | % of time CGM | | | | |
| | | > 140 mg/dL (%) (resc) | - | - | 9.6 [5.6, 12.8] |
| | | < 70 mg/dL (%) | 38.3 [34.6, 44.1] | 34.7 [30.7, 39.2] | 6.5 [3.0, 8.7] |
| | < 54 mg/dL (%) | | 27.0 [17.0, 30.4] | 21.6 [16.1, 26.4] | 0.1 [0.0, 0.3] |
| | Events with rescues (%) | | - | - | 96.7 [96.7, 96.7] |
| | Mean rescues (g) | | - | - | 27.2 [23.7, 31.0] |

Even with the additional carbohydrate intake, the controller achieved a time in hyperglycemia similar to the NoExComp, as shown in Figure 7. The mIMC increases the CGM mean to 4.28 mg/dL (CI:2.90 - 5.91) mg/dL on average; although statistically significant, this increase, concerning NoExComp, has a minor relevance from the clinical point of view. The rise in the %time above 140 mg/dL after the rescue carbohydrate suggestion is also permissible (Table 6).

**Claims**

1. Method for improving blood glucose control of a hybrid controller, eliminating meal and exercise announcement by substituting patient-initiated meal boluses of the hybrid controller by an automatic insulin correction signal without retuning of said hybrid controller, and incorporating rescue carbohydrates suggestion, the method comprising the steps of:

   - measuring a plasma glucose (G(t)) signal by means of a continuous glucose monitor (CGM);
   - calculating a glucose level ($\hat{G}(t)$) by using a glucose-insulin model (M) describing the effect of insulin and rescue carbohydrates on glucose;
   - computing a disturbance term d(t) as:

$$d(t) = \hat{G}(t) - G(t)$$

- generating a virtual signal $u_{IMC}(t)$, for mitigating the effect of $d(t)$ on the output, by means of an IMC filter $Q(s)$, defined as:

$$Q(s) = \frac{u_{IMC}(s)}{d(s)} = F(s) \cdot H^{-1}(s)$$

wherein s is the Laplace variable, $H(s)$ is given by the linearization of the model M with respect to insulin input, and filter $F(s)$ is defined so that the degree of the numerator of $Q(s)$ is lower than the degree of the denominator of $Q(s)$;
- converting the virtual signal $u_{IMC}(t)$ into three feedforward actions: insulin infusion, rescue carbohydrate suggestion, and insulin-on-board reduction by:

> - setting insulin $u_{ins}(t)$ to 0 if $u_{IMC}(t)$ is lower than a positive threshold $th_{ins}$;
> - matching $u_{ins}(t)$ to $u_{IMC}(t)$, to add insulin to the main controller, limited by an upper saturation threshold $th_{sat}$, if $u_{IMC}(t)$ is higher than $th_{ins}$, or subtract insulin from the main controller, when $u_{IMC}(t)$ is below the negative threshold $th_{resc}$;
> - converting the negative-valued insulin $u_{IMC}(t)$ into rescue carbohydrates suggestions ($u_{resc}$), then zeroing $u_{ins}(t)$ to avoid both types of control actions coupling each other if $u_{IMC}(t)$ is lower than $th_{resc}$, comprising conversion of negative insulin into rescue carbohydrate suggestions the steps of:

> > ○ generating a virtual unquantized carbohydrate signal, $u_{int}(t)$, integrating $u_{IMC}(t)$ in a sliding window of length $t_w$, as follows:

$$u_{int}(t) = -k_{resc} \int_{t-t_w}^{t} u_{IMC}^{*}(\tau)W(\tau)d\tau - \int_{t-t_w-T_s}^{t-T_s} u_{resc}(\tau)W(\tau)d\tau$$

> > using a variable $u_{IMC}^{*}$ instead of $u_{IMC}$ to avoid suggesting rescue carbohydrates when insulin inhibition may suffice, calculated as:

$$u_{IMC}^{*}(t) = \begin{cases} u_{IMC}(t) & \text{if} \quad u_{IMC}(t) \leq th_{resc} \\ 0 & \text{otherwise} \end{cases}$$

> > and where $T_s$ is the sampling time, $k_{resc}$ is a gain for converting "negative insulin" into rescue carbohydrate suggestions and $W(t^*)$ is a forgetting factor that attenuates the earlier values of $u_{IMC}^{*}(t)$ by using a monotone non-decreasing function $W(t^*)$, which has values
> > for $t^* \in [t - t_w, t]$, where t refers to the current time and $t - t_w$ the beginning of the sliding window, and so that $W(t)=1$
> > ○ calculating $u_{resc}(t)$ by:

$$u_{resc}(t) = \begin{cases} \left\lceil \frac{u_{int}(t)}{\widetilde{cho}} \right\rceil \cdot \widetilde{cho} & \text{if} & u_{int}(t) \geq 0.5\widetilde{cho} \text{ and} \\ & & G^{*}(t) \leq C_2 \text{ and } \Delta t_{resc} > C_1 \\ \widetilde{cho} & \text{if} & G^{*}(t) \leq C_3 \text{ and} \\ & & CGM(t) \leq C_2 \text{ and } \Delta t_{resc} > C_1 \\ 0 & & \text{otherwise} \end{cases}$$

> > wherein

$$\lceil \cdot \rceil$$

> > denotes the nearest integer operator, $\widetilde{cho}$ is the quantization level, $\Delta t_{resc}$ is the elapsed time between two consecutive rescue carbohydrate suggestions, $C_1$ is a parameter introduced as a delay for avoiding consecutive rescue carbohydrate suggestions, $C_2$ and $C_3$ are parameters related to mild and moderate hypoglycemia thresholds, and G*(t) is a glucose prediction.

2. Method according to claim 1, wherein the glucose-insulin model is the Identifiable Virtual Patient (IVP) defined by:

$$\dot{I}_{SC}(t) = -\frac{1}{\tau_1} I_{SC}(t) + \frac{\kappa}{\tau_1 C_I} u_T(t)$$

$$\dot{I}_P(t) = -\frac{1}{\tau_2} I_P(t) + \frac{1}{\tau_2} I_{SC}(t)$$

$$\dot{I}_{EFF}(t) = -p_2 I_{EFF}(t) + p_2 S_I I_P(t)$$

$$\dot{d}_1(t) = A_g^{resc} \cdot u_{resc}(t) - \frac{d_1(t)}{\tau_{resc}}$$

$$\dot{d}_2(t) = \frac{1}{\tau_{resc}} \left( d_1(t) - d_2(t) \right)$$

$$\dot{G}(t) = -GEZI \cdot G(t) - I_{EFF}(t) \cdot G(t) + EGP + \frac{d_2(t)}{V_g \tau_{resc}}$$

where $I_{SC}(t)$ and Ip(t) are the subcutaneous and plasma insulin concentrations ($\mu$U/mL), respectively, $I_{EFF}(t)$ represents the insulin effect, and G(t) is the plasma glucose concentration (mg/dL), the subcutaneous insulin infusion $u_T(t)$ ($\mu$U/min) and the rescue carbohydrate suggestion $u_{resc}(t)$ (mg/min) are defined, and a two-compartment model, with the glucose masses (mg) d1(t), d2(t) as states, models the rescue carbohydrates absorption, also, the parameters $\tau$1 and $\tau$2 (min) stand for the insulin absorption time constants, and p2 is the kinetic rate for insulin action, parameter $C_I$ denotes the insulin clearance (mL/min), $S_I$ represents the insulin sensitivity (mL/$\mu$U), EGP is the hepatic glucose production (mg/dL/min), GEZI corresponds to the glucose effectiveness at zero insulin (min$^{-1}$), parameter $\tau_{resc}$ is the time to the peak absorption of the rescue carbohydrate, $A_g^{resc}$ is the carbohydrate bioavailability, and $\kappa = 60 \cdot 10^{-6}$ is a conversion factor.

3. Method according to claim 2, where the linearized model H(s) is computed as:

$$H(s) := \frac{G(s)}{u_T(s)} = \frac{S_I G_0^2}{C_I EGP \left( \frac{1}{p_2} s + 1 \right) (\tau_1 s + 1)(\tau_2 s + 1) \left( \frac{G_0}{EGP} s + 1 \right)}$$

4. Method according to claim 1, wherein the monotone non-decreasing function W(t*), is represented by an exponential expression of the form:

$$W(t^*) = C_4 \cdot e^{-\frac{(t^* - t + t_w)\ln(C_4)}{t_w}}$$

for $t^* \in [t - t_w, t]$, where t refers to the current time and $t - t_w$ the beginning of the sliding window, with $C_4$ being a constant that states the level of attenuation of $u_{IMC}^*(t)$ and verifies $0 < C_4 \leq 1$; and $\ln(C_4)$ denotes the natural logarithm of $C_4$.

5. Method according to claim 1, wherein the sliding window, is given by the forgetting factor W(t*) defined as:

$$W(t^*) = e^{-2} \cdot e^{(t^*-t+60)/30}$$

6. Method according to claim 1, wherein the glucose prediction G*(t) is computed with the following linear extrapolation:

$$G^*(t) = CGM(t) + C_5 \cdot \frac{dCGM(t)}{dt}$$

wherein $C_5$ is a parameter fixing the time of the glucose prediction to 30 min.

7. Method according to claim 1, wherein the filter F(s) is defined as:

$$F(s) = \frac{k}{(\tau s + 1)^n}$$

where k is the gain of the filter, n is high enough so that the degree of the numerator of Q(s) is lower than the degree of the denominator of Q(s), and $\tau$ is a time constant which determines the aggressiveness of the filter.

8. Method according to claim 1, wherein, after a rescue carbohydrates suggestion, the switching logic:

- introduces a more strict limitation of the tolerated insulin-on-board of the main controller by:

  ∘ decreasing an upper limit of insulin-on-board in a given percentage to a percentage between 10% and 90% of its nominal value;
  ∘ adjusting a gain parameter in the controller so that infused insulin is decreased; or
  ∘ adjusting the cost of a receding horizon based controller so that infused insulin is decreased;

- zeroes $u_{ins}(t)$ during a predefined time $T_1$ following the last rescue carbohydrate suggestion; and
- restores the nominal values of insulin-on-board limitation and $u_{ins}(t)$ when CGM(t) is over a first threshold $Th_1$ and G*(t) over a second threshold $Th_2$.

9. Method according to claim 8, wherein the predefined time $T_1$ is in the range from 30 to 300 min, and the thresholds $Th_1$ and $Th_2$ are defined as: CGM(t) ≥ 140 mg/dL and G*(t) ≥ 180 mg/dL

10. Method according to claim 1, wherein the length of the sliding window $t_w$ is set to 60 min.

11. Method according to claim 1, wherein the time constant $\tau$ which determines the aggressiveness of the filter is set to two times the sampling time of the CGM.

12. Method according to claim 1, further comprising an optimization step for tunning the parameters k, $th_{ins}$, $th_{sat}$, $k_{resc}$, $th_{resc}$ by applying a cost defined as:

$$J_{sim} := J_{WAIR} + J_C$$

wherein:

- the term $J_{WAIR}$ penalizes the weighted areas of the CGM exceeding certain thresholds $g_{uu}$, $g_u$, $g_l$ and $g_{ll}$ as follows:

$$J_{WAIR} = a_{uu} \cdot \int_0^{Tsim} (G_{uu}(\tau) - g_{uu})\, d\tau + a_u \cdot \int_0^{Tsim} (G_u(\tau) - g_u)\, d\tau +$$

$$+ a_l \cdot \int_0^{Tsim} (g_l - G_l(\tau))\, d\tau + a_{ll} \cdot \int_0^{Tsim} (g_{ll} - G_{ll}(\tau))\, d\tau +$$

$$+ a_{resc} \cdot \int_0^{Tsim} (G_{resc}(\tau) - g_{resc})\, d\tau$$

wherein $T_{sim}$ is the simulation length, the parameters $a_{uu}$, $a_u$, $a_l$, $a_{ll}$, and, $a_{resc}$ are non-negative scalars defining the weight of each term, the thresholds are positive scalars defined so that $g_{ll} < g_{ll} < g_u < g_{uu}$, signals $G_{uu}(t)$, $G_u(t)$, $G_l(t)$, $G_{ll}(t)$ correspond to the CGM after being saturated to the enclosing thresholds as follows:

$$G_{uu}(t) := \begin{cases} g_{uu} & \text{if} & CGM(t) \leq g_{uu} \\ CGM(t) & \text{otherwise} \end{cases}$$

$$G_u(t) := \begin{cases} g_u & \text{if} & CGM(t) \leq g_u \\ g_{uu} & \text{if} & CGM(t) > g_{uu} \\ CGM(t) & \text{otherwise} \end{cases}$$

$$G_l(t) := \begin{cases} g_{ll} & \text{if} & CGM(t) < g_{ll} \\ g_l & \text{if} & CGM(t) \geq g_l \\ CGM(t) & \text{otherwise} \end{cases}$$

$$G_{ll}(t) := \begin{cases} g_{ll} & \text{if} & CGM(t) \geq g_{ll} \\ CGM(t) & \text{otherwise} \end{cases}$$

and wherein the last addend of $J_{WAIR}$ weights the glucose rebound after rescue carbohydrate suggestion time to better coordinate rescue carbohydrates suggestions and insulin doses, wherein signal $G_{resc}(t)$ represents the value of the CGM that overpasses a certain limit of the value of $g_{resc}$ in the first minutes before the value of $C_6$ after rescue carbohydrate suggestions, wherein if a meal occurred before $C_6$, $G_{resc}(t)$ was calculated until mealtime as defined in:

$$G_{resc}(t) = \begin{cases} CGM(t) & \text{if} & \begin{aligned} & (CGM(t) \geq g_{resc})\ and \\ & t \in [t_{resc}, \min(t_{resc} + C_6, t_{meal})] \end{aligned} \\ g_{resc} & \text{otherwise} \end{cases}$$

where $t_{resc}$ and $t_{meal}$ denote the rescue carbohydrates and meals times, respectively; and
- the second term of the cost function, $J_C$, constrains the shape and magnitude of the control actions as defined below:

$$J_C = b_{act} \cdot \max\left(\frac{n_{imc\_act}}{n_{meal}} - 1{,}0\right) + b_{meal\_resc} \cdot \sum_{i=1}^{n_{meal\_resc}} meal\_resc_i +$$

$$+ b_{ex\_resc} \cdot \max\left(\frac{\sum_{i=1}^{n_{ex\_resc}} ex\_resc_i}{C_7 n_{ex\_sessions}} - 1{,}0\right)$$

where:

◦ the first addend penalizes situations wherein the number of IMC activations in insulin mode (n_imc_act) is larger than the number of meals (n_meal) in the optimization scenario, wherein the parameter $b_{act}$ is a non-negative scalar weight of the addend;

◦ the second addend constrains the total amount of rescues given after meals, wherein the carbohydrate amount given at meal i is defined as meal_resc_i and n $_{meal\_resc}$ denotes the number of rescues given after meals, and the parameter b $_{meal\_resc}$ is a non-negative scalar weight of the addend; and

◦ the third addend penalizes situations wherein the amount of suggested carbohydrate per exercise event exceeds a certain value of $C_7$, wherein the carbohydrate amount suggested after exercise i is denoted as ex_resc_i, and n_ex_resc and n_ex_sessions define the number of exercise-related rescues and exercise events, respectively, being the parameter b $_{ex\_resc}$ a non-negative scalar weight of the addend.

13. Method according to claim 12, wherein the thresholds gresc, gll, gl, gu and guu are in the range from 40 to 400 mg/dL; $C_6$ in the calculation of Gresc(t) is in the range from 5 to 300 min; and the tolerable amount of suggested carbohydrate per exercise event in $J_C$, represented by $C_7$, is between 5 g and 100 g.

14. Add-on module for being incorporated to an artificial pancreas system comprising a calculation unit configured to carry out the steps of any of claims 1 to 13.

15. Artificial pancreas system for performing the method as defined in any of claims 1 to 13, comprising:

- a pump (3), for supplying insulin according to a coordinated control action ($u_c$);
- a continuous glucose monitor (2) for measuring the plasma glucose (G(t)) signal;
- a controller for determining the insulin level to be delivered, and
- an add-on module comprising a calculation unit configured to carry out the steps of any of claims 1 to 13.

**Patentansprüche**

1. Verfahren zur Verbesserung der Blutzuckerkontrolle eines Hybridsteuergeräts, wobei die Ankündigung von Mahlzeiten und körperlicher Betätigung entfällt, indem die vom Patienten eingeleiteten Mahlzeitenbolusse des Hybridsteuergeräts durch ein automatisches Insulinkorrektursignal ersetzt werden, ohne dass das Hybridsteuergerät neu eingestellt wird, und wobei ein Vorschlag für Notfall-Kohlenhydrate integriert wird; wobei das Verfahren die folgenden Schritte umfasst:

- Messen eines Plasmaglukose(G(t))-Signals mittels eines kontinuierlichen Glukosemonitors (CGM);
- Berechnen eines Glukosespiegels ($\hat{G}(t)$) unter Verwendung eines Glukose-Insulin-Modells (M), das die Wirkung von Insulin und Notfall-Kohlenhydraten auf die Glukose beschreibt;
- Berechnen eines Störterms d(t) als:

$$d(t) = \hat{G}(t) - G(t)$$

- Generieren eines virtuellen Signals u$_{IMC}$(t) zum Mindern der Wirkung von d(t) auf die Ausgabe mittels eines IMC-Filters Q(s), der wie folgt definiert ist:

$$Q(s) = \frac{u_{IMC}(s)}{d(s)} = F(s) \cdot H^{-1}(s)$$

wobei s die Laplace-Variable ist, H(s) durch die Linearisierung des Modells M in Bezug auf die Insulineingabe gegeben ist und der Filter F(s) so definiert ist, dass der Grad des Zählers von Q(s) niedriger ist als der Grad des Nenners von Q(s);

- Umwandeln des virtuellen Signals u$_{IMC}$(t) in drei Vorsteuerungsmaßnahmen: Insulininfusion, Vorschlag für Notfall-Kohlenhydrate und Reduzierung des aktiven Insulinspiegels durch:

- Setzen von Insulin u$_{ins}$(t) auf 0, falls u$_{IMC}$(t) kleiner als ein positiver Schwellenwert th$_{ins}$ ist;
- Abgleichen von u$_{ins}$(t) mit u$_{IMC}$(t), um Insulin zum Hauptsteuergerät hinzuzufügen, begrenzt durch einen oberen Sättigungsschwellenwert th$_{sat}$, falls u$_{IMC}$(t) höher als th$_{ins}$ ist, oder Insulin vom Hauptsteuergerät zu subtrahieren, wenn u$_{IMC}$(t) unter dem negativen Schwellenwert th$_{Notf}$ liegt;
- Umwandeln des negativwertigen Insulins u$_{IMC}$(t) in Vorschläge für Notfall-Kohlenhydrate (u$_{Notf}$), an-

schließendes Nullsetzen von $u_{ins}(t)$, um eine gegenseitige Kopplung beider Steuerungsmaßnahmen zu vermeiden, falls $u_{IMC}(t)$ kleiner als $th_{Notf}$ ist, wobei die Umwandlung von negativem Insulin in Vorschläge für Notfall-Kohlenhydrate die folgenden Schritte umfasst:

◦ Generieren eines virtuellen, unquantisierten Kohlenhydratsignals, $u_{int}(t)$, durch Integrieren von $u_{IMC}(t)$ in einem gleitenden Fenster der Länge $t_w$ wie folgt:

$$u_{int}(t) = -k_{Notf}\int_{t-t_w}^{t} u_{IMC}^{*}(\tau)W(\tau)d\tau - \int_{t-t_w-T_s}^{t-T_s} u_{Notf}(\tau)W(\tau)d\tau$$

unter Verwendung einer variablen $u_{IMC}^{*}$ anstelle von $u_{IMC}$, um den Vorschlag für Notfall-Kohlenhydrate zu vermeiden, wenn eine Insulinhemmung ausreichen könnte, berechnet als:

$$u_{IMC}^{*}(t) = \begin{cases} u_{IMC}(t) & \text{falls} & u_{IMC}(t) \leq th_{Notf} \\ 0 & \text{anderenfalls} \end{cases}$$

und wobei $T_s$ die Abtastzeit ist, $k_{Notf}$ eine Verstärkung für das Umwandeln von "negativem Insulin" in Vorschläge für Notfall-Kohlenhydrate ist und $W(t^*)$ ein Vergessensfaktor ist, der die früheren Werte von

$u_{IMC}^{*}(t)$ unter Verwendung einer monoton nicht abnehmenden Funktion $W(t^*)$ abschwächt, die Werte für $t^* \in [t - t_w, t]$ aufweist, wobei $t$ die aktuelle Zeit und $t - t_w$ den Beginn des gleitenden Fensters bezeichnet, und so dass $W(t)=1$
o Berechnen von $u_{Notf}(t)$ durch:

$$u_{Notf}(t) = \begin{cases} \left\lfloor \dfrac{u_{int}(t)}{\widetilde{cho}} \right\rfloor \cdot \widetilde{cho} & \text{falls} & u_{int}(t) \geq 0{,}5\widetilde{cho} \text{ und } \\ & & G^*(t) \leq C_2 \text{ und } \Delta t_{Notf} > C_1 \\ \widetilde{cho} & \text{falls} & G^*(t) \leq C_3 \text{ und } \\ & & CGM(t) \leq C_2 \text{ und } \Delta t_{Notf} > C_1 \\ 0 & \text{anderenfalls} \end{cases}$$

wobei

$$\lfloor \cdot \rfloor$$

den Operator für die nächste ganze Zahl bezeichnet, $\widetilde{cho}$ die Quantisierungsstufe ist, $\Delta t_{Notf}$ die verstrichene Zeit zwischen zwei aufeinanderfolgenden Vorschlägen für Notfall-Kohlenhydrate ist, $C_1$ ein Parameter ist, der als Verzögerung eingeführt wird, um aufeinanderfolgende Vorschläge für Notfall-Kohlenhydrate zu vermeiden, $C_2$ und $C_3$ Parameter sind, die mit Schwellenwerten für leichte und mittelschwere Hypoglykämie zusammenhängen, und $G^*(t)$ eine Glukosevorhersage ist.

2. Verfahren nach Anspruch 1, wobei das Glukose-Insulin-Modell der Identifizierbare Virtuelle Patient (IVP) ist, der wie folgt definiert ist:

$$\dot{I}_{SC}(t) = -\frac{1}{\tau_1}I_{SC}(t) + \frac{\kappa}{\tau_1 C_I}u_T(t)$$

$$\dot{I}_P(t) = -\frac{1}{\tau_2}I_P(t) + \frac{1}{\tau_2}I_{SC}(t)$$

$$\dot{I}_{EFF}(t) = -p_2 I_{EFF}(t) + p_2 S_I I_P(t)$$

$$\dot{d}_1(t) = A_g^{Notf} \cdot u_{Notf}(t) - \frac{d_1(t)}{\tau_{Notf}}$$

$$\dot{d}_2(t) = \frac{1}{\tau_{Notf}}\big(d_1(t) - d_2(t)\big)$$

$$\dot{G}(t) = -GEZI \cdot G(t) - I_{EFF}(t) \cdot G(t) + EGP + \frac{d_2(t)}{V_g \tau_{Notf}}$$

wobei $I_{SC}(t)$ und $I_P(t)$ jeweils die subkutane und die Plasmainsulinkonzentration ($\mu$U/ml) sind, $I_{EFF}(t)$ die Insulinwirkung darstellt und G(t) die Plasmaglukosekonzentration (mg/dl) ist, wobei die subkutane Insulininfusion $u_T$ (t) ($\mu$U/min) und der Vorschlag für Notfall-Kohlenhydrate $u_{Notf}(t)$ (mg/min) definiert sind, und ein Zwei-Kompartiment-Modell mit den Glukosemassen (mg) d1(t) und d2(t) als Zuständen die Absorption der Notfall-Kohlenhydrate modelliert, wobei ferner die Parameter $\tau1$ und $\tau2$ (min) für die Insulinabsorptionszeitkonstanten stehen und p2 die kinetische Rate der Insulinwirkung ist, der Parameter $C_I$ die Insulin-Clearance (ml/min) bezeichnet, $S_I$ die Insulinsensitivität (ml/$\mu$U) darstellt, EGP die hepatische Glukoseproduktion (mg/dl/min) ist, GEZI der Glukoseeffektivität bei Null-Insulin (min$^{-1}$) entspricht, der Parameter $\tau_{Notf}$ die Zeit bis zur maximalen Absorption der Notfall-Kohlenhydrate ist, $A_g^{Notf}$ die Bioverfügbarkeit der Kohlenhydrate ist und $\kappa = 60 \cdot 10^{-6}$ ein Umrechnungsfaktor ist.

3. Verfahren nach Anspruch 2, wobei das linearisierte Modell H(s) wie folgt berechnet wird:

$$H(s) := \frac{G(s)}{u_T(s)} = \frac{S_I G_0^2}{C_I EGP \left(\frac{1}{p_2}s + 1\right)(\tau_1 s + 1)(\tau_2 s + 1)\left(\frac{G_0}{EGP}s + 1\right)}$$

4. Verfahren nach Anspruch 1, wobei die monoton nicht abnehmende Funktion W(t*) durch einen Exponentialausdruck der folgenden Form dargestellt wird:

$$W(t^*) = C_4 \cdot e^{-\frac{(t^*-t+t_w)\ln(C_4)}{t_w}}$$

für t* $\in$ [t - $t_w$, t], wobei t die aktuelle Zeit und t - $t_w$ den Beginn des gleitenden Fensters bezeichnet, wobei $C_4$ eine Konstante ist, die den Abschwächungsgrad von $u_{IMC}^*$ (t) angibt und $0 < C_4 \leq 1$ verifiziert; und ln $(C_4)$ den natürlichen Logarithmus von $C_4$ bezeichnet.

5. Verfahren nach Anspruch 1, wobei das gleitende Fenster durch den Vergessensfaktor W(t*) gegeben ist, der wie folgt definiert ist:

$$W(t^*) = e^{-2} \cdot e^{(t^*-t+60)/30}$$

6. Verfahren nach Anspruch 1, wobei die Glukosevorhersage G*(t) mit der folgenden linearen Extrapolation berechnet wird:

$$G^*(t) = CGM(t) + C_5 \cdot \frac{dCGM(t)}{dt}$$

wobei $C_5$ ein Parameter ist, der die Zeit der Glukosevorhersage auf 30 min festlegt.

7.  Verfahren nach Anspruch 1, wobei der Filter F(s) wie folgt definiert ist:

$$F(s) = \frac{k}{(\tau s + 1)^n}$$

wobei k die Verstärkung des Filters ist, n hoch genug ist, sodass der Grad des Zählers von Q(s) niedriger ist als der Grad des Nenners von Q(s), und $\tau$ eine Zeitkonstante ist, die die Aggressivität des Filters bestimmt.

8.  Verfahren nach Anspruch 1, wobei nach einem Vorschlag für Notfall-Kohlenhydrate die Schaltlogik:

    - eine strengere Begrenzung des tolerierten aktiven Insulins des Hauptsteuergeräts einführt durch:

        ◦ Verringern der Obergrenze für das aktive Insulin in einem gegebenen Prozentsatz auf einen Prozentsatz zwischen 10 % und 90 % ihres Nennwerts;
        ◦ Anpassen eines Verstärkungsparameters im Steuergerät, um das infundierte Insulin zu verringern; oder
        ◦ Anpassen der Kosten eines auf den zurückgehenden Horizont basierenden Steuergeräts, um das infundierte Insulin zu verringern;

    - $u_{ins}(t)$ während einer vordefinierten Zeit $T_1$ nach dem letzten Vorschlag für Notfall-Kohlenhydrate auf Null setzt; und
    - die Nennwerte der Begrenzung des aktiven Insulins und $u_{ins}(t)$ wieder herstellt, wenn CGM(t) über einen ersten Schwellenwert $Th_1$ und G*(t) über einen zweiten Schwellenwert $Th_2$ liegt.

9.  Verfahren nach Anspruch 8, wobei die vordefinierte Zeit $T_1$ im Bereich von 30 bis 300 min liegt und die Schwellenwerte $Th_1$ und $Th_2$ wie folgt definiert sind: CGM(t) $\geq$ 140 mg/dl und G*(t) $\geq$ 180 mg/dl

10. Verfahren nach Anspruch 1, wobei die Länge des gleitenden Fensters $t_w$ auf 60 min eingestellt ist.

11. Verfahren nach Anspruch 1, wobei die Zeitkonstante $\tau$, die die Aggressivität des Filters bestimmt, auf das Zweifache der Abtastzeit des CGM eingestellt ist.

12. Verfahren nach Anspruch 1, ferner umfassend einen Optimierungsschritt zum Einstellen der Parameter k, $th_{ins}$, $th_{sät}$, $k_{Notf}$, $th_{Notf}$ durch Anwenden eines wie folgt definierten Kostenfaktors:

$$J_{sim} := J_{WAIR} + J_C$$

wobei:

    - der Term $J_{WAIR}$ die gewichteten Bereiche des CGM bestraft, die gewisse Schwellenwerte $g_{uu}$, $g_u$, $g_l$ und $g_{ll}$ überschreiten, wie folgt:

$$J_{WAIR} = a_{uu} \cdot \int_0^{Tsim} (G_{uu}(\tau) - g_{uu})d\tau + a_u \cdot \int_0^{Tsim} (G_u(\tau) - g_u)d\tau +$$
$$+ a_l \cdot \int_0^{Tsim} (g_l - G_l(\tau))d\tau + a_{ll} \cdot \int_0^{Tsim} (g_{ll} - G_{ll}(\tau))d\tau +$$
$$+ a_{Notf} \cdot \int_0^{Tsim} (G_{Notf}(\tau) - g_{Notf})d\tau$$

wobei $T_{sim}$ die Simulationslänge ist, die Parameter $a_{uu}$, $a_u$, $a_l$, $a_{ll}$ und $a_{Notf}$ nicht-negative Skalare sind, die das Gewicht jedes Terms definieren, die Schwellenwerte positive Skalare sind, die so definiert sind, dass $g_{ll} < g_l < g_u < g_{uu}$, wobei die Signale $G_{uu}(t)$, $G_u(t)$, $G_l(t)$, $G_{ll}(t)$ dem CGM nach Sättigung bis zu den umschließenden Schwellenwerten wie folgt entsprechen:

$$G_{uu}(t) := \begin{cases} g_{uu} & \text{falls} & CGM(t) \le g_{uu} \\ CGM(t) & \text{anderenfalls} \end{cases}$$

$$G_u(t) := \begin{cases} g_u & \text{falls} & CGM(t) \le g_u \\ g_{uu} & \text{falls} & CGM(t) > g_{uu} \\ CGM(t) & \text{anderenfalls} \end{cases}$$

$$G_l(t) := \begin{cases} g_{ll} & \text{falls} & CGM(t) < g_{ll} \\ g_l & \text{falls} & CGM(t) \ge g_l \\ CGM(t) & \text{anderenfalls} \end{cases}$$

$$G_{ll}(t) := \begin{cases} g_{ll} & \text{falls} & CGM(t) \ge g_{ll} \\ CGM(t) & \text{anderenfalls} \end{cases}$$

und wobei der letzte Summand von $J_{WAIR}$ den Glukose-Rebound nach der Zeit des Vorschlags für Notfall-Kohlenhydrate gewichtet, um die Vorschläge für Notfall-Kohlenhydrate und die Insulindosen besser zu koordinieren, wobei das Signal $G_{Notf}(t)$ den Wert des CGM darstellt, der eine gewisse Grenze des Wertes von $g_{Notf}$ in den ersten Minuten vor dem Wert von $C_6$ nach den Vorschlägen für Notfall-Kohlenhydrate überschreitet, wobei, falls vor $C_6$ eine Mahlzeit stattfand, $G_{Notf}(t)$ bis zum Zeitpunkt der Mahlzeit gemäß folgender Definition berechnet wurde:

$$G_{Notf}(t) = \begin{cases} CGM(t) & \text{falls} & \begin{array}{l}\left(CGM(t) \ge g_{Notf}\right) \text{ und} \\ t \in \left[t_{Notf}, \min\left(t_{Notf} + C_6, t_{Mahlzeit}\right)\right]\end{array} \\ g_{Notf} & \text{anderenfalls} \end{cases}$$

wobei $t_{Notf}$ und $t_{Mahlzeit}$ jeweils die Zeiten für die Notfall-Kohlenhydrate und die Mahlzeiten bezeichnen; und
- der zweite Term der Kostenfunktion, Jc, die Form und das Ausmaß der Steuerungsmaßnahmen wie unten definiert beschränkt:

$$J_C = b_{Akt} \cdot \max\left(\frac{n_{imc\_Akt}}{n_{Mahlzeit}} - 1{,}0\right) + b_{Mahlzeit\_Notf}$$
$$\cdot \sum_{i=1}^{n_{Mahlzeit\_Notf}} Mahlzeit\_Notf_i +$$
$$+ b_{Tr\_Notf} \cdot \max\left(\frac{\sum_{i=1}^{n_{Tr\_Notf}} Tr\_Notf_i}{C_7 n_{Tr\_Einheiten}} - 1{,}0\right)$$

wo:

∘ der erste Summand Situationen bestraft, in denen die Anzahl der IMC-Aktivierungen im Insulinmodus (n_imc_Akt) größer ist als die Anzahl der Mahlzeiten (n_Mahlzeit) im Optimierungsszenario, wobei der Parameter $b_{Akt}$ ein nicht-negatives Skalargewicht des Summanden ist;
∘ der zweite Summand die Gesamtmenge der nach den Mahlzeiten verabreichten Notfallmaßnahmen beschränkt, wobei die bei Mahlzeit i verabreichte Kohlenhydratmenge als Mahlzeit_Notf_i definiert ist und n_Mahlzeit_Notf die Anzahl der nach den Mahlzeiten verabreichten Notfallmaßnahmen bezeichnet und der Parameter b_Mahlzeit_Notf ein nicht-negatives Skalargewicht des Summanden ist; und
∘ der dritte Summand Situationen bestraft, in denen die vorgeschlagene Kohlenhydratmenge pro Trainingsereignis einen gewissen Wert von $C_7$ überschreitet, wobei die nach dem Training i vorgeschlagene Kohlenhydratmenge mit Tr_Notf_i bezeichnet wird und n_Tr_Notf und n_Tr_Einheiten jeweils die Anzahl der trainingsbedingten Rettungsmaßnahmen und Trainingsereignisse definieren, wobei der Parameter b$_{Tr\_Notf}$ ein nicht-negatives Skalargewicht des Summanden ist.

13. Verfahren nach Anspruch 12, wobei die Schwellenwerte gNotf, gll, gl, gu und guu im Bereich von 40 bis 400 mg/dl liegen; $C_6$ bei der Berechnung von GNotf(t) im Bereich von 5 bis 300 min liegt; und die tolerierbare Menge an vorgeschlagenen Kohlenhydraten pro Trainingsereignis in Jc, dargestellt durch $C_7$, zwischen 5 g und 100 g liegt.

14. Zusatzmodul zur Integration in ein künstliches Pankreassystem, umfassend eine Berechnungseinheit, die so konfiguriert ist, dass sie die Schritte nach einem der Ansprüche 1 bis 13 durchführt.

15. Künstliches Pankreassystem zum Durchführen des in einem der Ansprüche 1 bis 13 definierten Verfahrens, umfassend:

   - eine Pumpe (3) zum Zuführen von Insulin gemäß einer koordinierten Steuerungsmaßnahme ($u_c$);
   - einen kontinuierlichen Glukosemonitor (2) zum Messen des Plasmaglukose(G(t))-Signals;
   - ein Steuergerät zum Bestimmen der abzugebenden Insulinmenge, und
   - ein Zusatzmodul, umfassend eine Berechnungseinheit, die so konfiguriert ist, dass sie die Schritte nach einem der Ansprüche 1 bis 13 durchführt.

## Revendications

1. Procédé d'amélioration du contrôle de la glycémie d'un contrôleur hybride, éliminant des annonces de repas et d'exercice en remplaçant les bolus de repas initiés par le patient du contrôleur hybride par un signal de correction automatique d'insuline sans réajustement dudit contrôleur hybride, et intégrant une suggestion de glucides de secours, le procédé comprenant les étapes consistant à :

   - la mesure d'un signal de glucose plasmatique (G(t)) au moyen d'un moniteur continu de glucose (CGM) ;
   - le calcul d'un niveau de glucose ($\hat{G}(t)$) en utilisant un modèle glucose-insuline (M) décrivant l'effet de l'insuline et des glucides de secours sur le glucose ;
   - le calcul d'un terme de perturbation d(t) comme :

$$d(t) = \hat{G}(t) - G(t)$$

   - la génération d'un signal virtuel u$_{IMC}$(t), pour atténuer l'effet de d(t) sur la sortie, au moyen d'un filtre IMC Q(s), défini comme :

$$Q(s) = \frac{u_{IMC}(s)}{d(s)} = F(s) \cdot H^{-1}(s)$$

   où s est la variable de Laplace, H(s) est donné par la linéarisation du modèle M par rapport à l'entrée d'insuline, et le filtre F(s) est défini de sorte que le degré du numérateur de Q(s) soit inférieur au degré du dénominateur de Q(s) ;
   - la conversion du signal virtuel u$_{IMC}$(t) en trois actions anticipatives : perfusion d'insuline, suggestion de glucides de secours et réduction de l'insuline active par :

   - le réglage de l'insuline u$_{ins}$(t) à 0 si u$_{IMC}$(t) est inférieur à un seuil positif th$_{ins}$ ;
   - le fait de mettre en correspondance u$_{ins}$(t) à u$_{IMC}$(t), pour ajouter de l'insuline au contrôleur principal, limité par un seuil de saturation supérieur th$_{sat}$, si u$_{IMC}$(t) est supérieur à th$_{ins}$, ou soustraire l'insuline du contrôleur principal, lorsque u$_{IMC}$(t) est en dessous du seuil négatif th$_{sec}$ ;
   - la conversion de l'insuline à valeur négative u$_{IMC}$(t) en suggestions de glucides de secours (u$_{sec}$), puis la mise à zéro de u$_{ins}$(t) pour éviter que les deux types d'actions de contrôle ne se couplent si u$_{IMC}$(t) est inférieur à th$_{sec}$, comprenant la conversion de l'insuline négative en suggestions de glucides de secours, les étapes consistant à :

○la génération d'un signal glucidique virtuel non quantifié, $u_{int}(t)$, intégrant $u_{IMC}(t)$ dans une fenêtre glissante de longueur $t_w$, comme suit :

$$u_{int}(t) = -k_{sec} \int_{t-t_w}^{t} u_{IMC}^*(\tau)W(\tau)d\tau - \int_{t-t_w-T_s}^{t-T_s} u_{sec}(\tau)W(\tau)d\tau$$

utilisant une variable $u_{IMC}^*$ au lieu de $u_{IMC}$ pour éviter de suggérer des glucides de secours lorsque l'inhibition de l'insuline peut suffire, calculé comme :

$$u_{IMC}^*(t) = \begin{cases} u_{IMC}(t) & \text{si} \quad u_{IMC}(t) \leq th_{sec} \\ 0 & \text{sinon} \end{cases}$$

et où $T_s$ est le temps d'échantillonnage, $k_{sec}$ est un gain pour convertir « l'insuline négative » en suggestions de glucides de secours et $W(t^*)$ est un facteur d'oubli qui atténue les valeurs précédentes de $u_{IMC}^*$ (t) en utilisant une fonction monotone non décroissante $W(t^*)$, qui a des valeurs pour $t^* \in [t - t_w, t]$, où t désigne l'heure actuelle et $t - t_w$ le début de la fenêtre glissante, et de sorte que $W(t) = 1$

○ le calcul de $u_{sec}(t)$ par :

$$u_{sec}(t) = \begin{cases} \left| \left\lceil \dfrac{u_{int}(t)}{\widetilde{cho}} \right\rfloor \right| \cdot \widetilde{cho} & \text{si} & \begin{array}{l} u_{int}(t) \geq 0.5\widetilde{cho} \text{ et} \\ G^*(t) \leq C_2 \text{ et } \Delta t_{sec} > C_1 \end{array} \\ \widetilde{cho} & \text{si} & \begin{array}{l} G^*(t) \leq C_3 \text{ et} \\ CGM(t) \leq C_2 \text{ et } \Delta t_{sec} > C_1 \end{array} \\ 0 & \text{sinon} \end{cases}$$

où

$$\lfloor \cdot \rceil$$

dénote l'opérateur entier le plus proche, $\widetilde{cho}$ est le niveau de quantification, $\Delta t_{sec}$ est le temps écoulé entre deux suggestions consécutives de glucides de secours, $C_1$ est un paramètre introduit comme un délai pour éviter des suggestions consécutives de glucides de secours, $C_2$ et $C_3$ sont des paramètres relatifs aux seuils d'hypoglycémie légère et modérée, et $G^*(t)$ est une prédiction de glucose.

2. Procédé selon la revendication 1, dans lequel le modèle glucose-insuline est le patient virtuel identifiable (PVI) défini par :

$$\dot{I}_{SC}(t) = -\frac{1}{\tau_1}I_{SC}(t) + \frac{\kappa}{\tau_1 C_I}u_T(t)$$

$$\dot{I}_P(t) = -\frac{1}{\tau_2}I_P(t) + \frac{1}{\tau_2}I_{SC}(t)$$

$$\dot{I}_{EFF}(t) = -p_2 I_{EFF}(t) + p_2 S_I I_P(t)$$

$$\dot{d}_1(t) = A_g^{sec} \cdot u_{sec}(t) - \frac{d_1(t)}{\tau_{sec}}$$

$$\dot{\mathrm{d}}_2(t) = \frac{1}{\tau_{\mathrm{sec}}}\big(\mathrm{d}_1(t) - \mathrm{d}_2(t)\big)$$

$$\dot{G}(t) = -GEZI \cdot G(t) - I_{EFF}(t) \cdot G(t) + EGP + \frac{d_2(t)}{V_g \tau_{sec}}$$

où $I_{SC}(t)$ et $I_P(t)$ sont les concentrations d'insuline sous-cutanée et plasmatique ($\mu$U/ml), respectivement, $I_{EFF}(t)$ représente l'effet de l'insuline, et G(t) est la concentration de glucose plasmatique (mg/dl), la perfusion sous-cutanée d'insuline $u_T$ (t) ($\mu$U/min) et la suggestion de glucides de secours $u_{sec}$(t) (mg/min) sont définies, et un modèle à deux compartiments, avec les masses de glucose (mg) d1(t), d2(t) comme états, modèles l'absorption des glucides de secours, également, les paramètres $\tau1$ et $\tau2$ (min) représentent les constantes de temps d'absorption de l'insuline, et p2 est le taux cinétique de l'action de l'insuline, le paramètre $C_I$ dénote la clairance de l'insuline (ml/min), $S_I$ représente la sensibilité à l'insuline (ml/$\mu$U), EGP correspond à la production hépatique de glucose (mg/dl/min), GEZI correspond à l'efficacité du glucose à zéro insuline (min$^{-1}$), le paramètre $\tau_{sec}$ est le temps nécessaire pour atteindre le pic d'absorption du glucide de secours, $A_g^{sec}$ est la biodisponibilité des glucides, et $\kappa = 60 \cdot 10^{-6}$ est un facteur de conversion.

3. Procédé selon la revendication 2, où le modèle linéarisé H(s) est calculé comme suit :

$$H(s) := \frac{G(s)}{u_T(s)} = \frac{S_I G_0^2}{C_I EGP \left(\frac{1}{p_2} s + 1\right)(\tau_1 s + 1)(\tau_2 s + 1)\left(\frac{G_0}{EGP} s + 1\right)}$$

4. Procédé selon la revendication 1, dans lequel la fonction monotone non décroissante W(t*), est représentée par une expression exponentielle de la forme :

$$W(t^*) = C_4 \cdot e^{-\frac{(t^*-t+t_w)\ln(C_4)}{t_w}}$$

pour $t^* \in [t - t_w, t]$, où t désigne le temps actuelle et $t - t_w$ le début de la fenêtre glissante, avec $C_4$ étant une constante qui indique le niveau d'atténuation de $u_{\mathrm{IMC}}^*$ (t) et vérifie $0 < C_4 \le 1$ ; et ln $(C_4)$ dénote le logarithme naturel de $C_4$.

5. Procédé selon la revendication 1, dans lequel la fenêtre coulissante est donné par le facteur d'oubli W(t*) défini comme :

$$W(t^*) = e^{-2} \cdot e^{(t^*-t+60)/30}$$

6. Procédé selon la revendication 1, dans lequel la prédiction du glucose G*(t) est calculée avec l'extrapolation linéaire suivante :

$$G^*(t) = CGM(t) + C_5 \cdot \frac{dCGM(t)}{dt}$$

dans lequel $C_5$ est un paramètre fixant le temps de prédiction du glucose à 30 min.

7. Procédé selon la revendication 1, dans lequel le filtre F(s) est défini comme :

$$F(s) = \frac{k}{(\tau s + 1)^n}$$

où k est le gain du filtre, n est suffisamment élevé de sorte que le degré du numérateur de Q(s) est inférieur au degré du dénominateur de Q(s), et $\tau$ est une constante de temps qui détermine l'agressivité du filtre.

8. Procédé selon la revendication 1, dans lequel, après une suggestion de glucides de secours, la logique de commutation :

   - introduit une limitation plus stricte de l'insuline active tolérée du contrôleur principal en :

     ∘ diminuant une limite supérieure d'insuline active par un pourcentage donné à un pourcentage compris entre 10 % et 90 % de sa valeur nominale ;
     ∘ ajustant un paramètre de gain dans le contrôleur afin de diminuer l'insuline perfusée ; ou
     ∘ ajustant le coût d'un contrôleur à base d'horizon fuyant afin de réduire l'insuline perfusée ;

   - met à zéro $u_{ins}(t)$ pendant un temps prédéfini $T_1$ suivant la dernière suggestion de glucides de secours ; et
   - rétablit les valeurs nominales de la limitation de l'insuline active et de $u_{ins}(t)$ lorsque CGM(t) dépasse un premier seuil $Th_1$ et G*(t) dépasse un second seuil $Th_2$.

9. Procédé selon la revendication 8, dans lequel le temps prédéfini $T_1$ est compris dans la plage entre 30 et 300 min, et les seuils $Th_1$ et $Th_2$ sont définis comme : CGM(t) ≥ 140 mg/dl et G*(t) ≥ 180 mg/dl

10. Procédé selon la revendication 1, dans lequel la longueur de la fenêtre coulissante $t_w$ est fixée à 60 min.

11. Procédé selon la revendication 1, dans lequel la constante de temps $\tau$ qui détermine l'agressivité du filtre est fixée à deux fois le temps d'échantillonnage du CGM.

12. Procédé selon la revendication 1, comprenant en outre une étape d'optimisation pour le réglage des paramètres k, $th_{ins}$, $th_{sat}$, $k_{sec}$, $th_{sec}$ en appliquant un coût défini comme :

$$J_{sim} := J_{WAIR} + J_C$$

dans lequel :

- le terme $J_{WAIR}$ pénalise les zones pondérées du CGM dépassant certains seuils $g_{uu}, g_u, g_l$ et $g_{ll}$ comme suit :

$$J_{WAIR} = a_{uu} \cdot \int_0^{Tsim} (G_{uu}(\tau) - g_{uu})d\tau + a_u \cdot \int_0^{Tsim} (G_u(\tau) - g_u)d\tau +$$
$$+ a_l \cdot \int_0^{Tsim} (g_l - G_l(\tau))d\tau + a_{ll} \cdot \int_0^{Tsim} (g_{ll} - G_{ll}(\tau))d\tau +$$
$$+ a_{sec} \cdot \int_0^{Tsim} (G_{sec}(\tau) - g_{sec})d\tau$$

dans lequel $T_{sim}$ est la longueur de la simulation, les paramètres $a_{uu}$, $a_u$, $a_l$, $a_{ll}$, et, $a_{sec}$ sont des scalaires non négatifs définissant le poids de chaque terme, les seuils sont des scalaires positifs définis de sorte que $g_{ll} < g_l < g_u < g_{uu}$, les signaux $G_{uu}(t)$, $G_u(t)$, $G_i(t)$, $G_{ll}(t)$ correspondent au CGM après avoir été saturé aux seuils englobants comme suit :

$$G_{uu}(t) := \begin{cases} g_{uu} & \text{si} \quad CGM(t) \leq g_{uu} \\ CGM(t) & \text{sinon} \end{cases}$$

$$G_u(t) := \begin{cases} g_u & \text{si} \quad CGM(t) \leq g_u \\ g_{uu} & \text{si} \quad CGM(t) > g_{uu} \\ CGM(t) & \text{sinon} \end{cases}$$

$$G_l(t) := \begin{cases} g_{ll} & \text{si} & CGM(t) < g_{ll} \\ g_l & \text{si} & CGM(t) \geq g_l \\ CGM(t) & \text{sinon} \end{cases}$$

$$G_{ll}(t) := \begin{cases} g_{ll} & \text{si} & CGM(t) \geq g_{ll} \\ CGM(t) & \text{sinon} \end{cases}$$

et dans lequel le dernier cumulateur de $J_{WAIR}$ pondère le rebond de glucose après le temps de suggestion de glucides de secours afin de mieux coordonner les suggestions de glucides de secours et les doses d'insuline, dans lequel le signal $G_{sec}(t)$ représente la valeur du CGM qui dépasse une certaine limite de la valeur de $g_{sec}$ dans les premières minutes avant la valeur de $C_6$ après les suggestions de glucides de secours, dans lequel si un repas a eu lieu avant $C_6$, $G_{sec}(t)$ a été calculé jusqu'à l'heure du repas tel que défini dans :

$$G_{sec}(t) = \begin{cases} CGM(t) & \text{si} & \begin{array}{l}(CGM(t) \geq g_{sec}) \text{ et} \\ t \in [t_{sec}, \min(t_{sec} + C_6, t_{repas})]\end{array} \\ g_{sec} & \text{sinon} \end{cases}$$

où $t_{sec}$ et $t_{repas}$ dénotent les glucides de secours et les heures des repas, respectivement ; et
- le second terme de la fonction de coût, Jc, contraint la forme et l'amplitude des actions de contrôle telles que définies ci-dessous :

$$J_C = b_{act} \cdot \max\left(\frac{n_{imc\_act}}{n_{repas}} - 1.0\right) + b_{repas\_sec} \cdot \sum_{i=1}^{n_{repas\_sec}} repas\_sec_i +$$
$$+ b_{ex\_sec} \cdot \max\left(\frac{\sum_{i=1}^{n_{ex\_sec}} ex\_sec_i}{C_7 n_{ex\_sessions}} - 1.0\right)$$

où :

∘ le premier cumulateur pénalise les situations dans lesquelles le nombre d'activations IMC en mode insuline (n_imc_act) est supérieur au nombre de repas (n_repas) dans le scénario d'optimisation, dans lequel le paramètre $b_{act}$ est un poids scalaire non négatif du cumulateur ;
∘ le deuxième cumulateur contraint le nombre total de secours administrés après les repas, dans lequel la quantité de glucides donnée au repas i est définie comme repas_sec_i et n _repas_sec dénote le nombre de secours donnés après les repas, et le paramètre b _repas_sec est un poids scalaire non négatif du cumulateur ; et
∘ le troisième cumulateur pénalise les situations dans lesquelles la quantité de glucides suggérée par événement d'exercice dépasse une certaine valeur de C7, dans lequel la quantité de glucides suggérée après l'exercice i est dénotée par ex_sec_i, et n_ex_sec et n_ex_sessions définissent le nombre de secours liés à l'exercice et d'événements d'exercice, respectivement, le paramètre b _ex_sec étant un poids scalaire non négatif du cumulateur.

13. Procédé selon la revendication 12, dans lequel les seuils gsec, gll, gl, gu et guu sont compris dans la plage entre 40 et 400 mg/dl ; $C_6$ dans le calcul de Gsec(t) est compris dans la plage entre 5 et 300 min ; et la quantité tolérable de glucides suggérée par événement d'exercice dans Jc, représenté par $C_7$, se situe entre 5 g et 100 g.

14. Module complémentaire destiné à être intégré à un système de pancréas artificiel comprenant une unité de calcul conçue pour exécuter les étapes de l'une quelconque des revendications 1 à 13.

15. Système de pancréas artificiel pour mettre en œuvre le procédé tel que défini dans l'une quelconque des revendications 1 à 13, comprenant :

- une pompe (3), pour fournir de l'insuline selon une action de contrôle coordonnée ($u_c$) ;
- un moniteur continu de glucose (2) pour mesurer le signal du glucose plasmatique (G(t)) ;
- un contrôleur pour déterminer le niveau d'insuline à administrer, et
- un module complémentaire comprenant une unité de calcul conçue pour exécuter les étapes de l'une quelconque des revendications 1 à 13.

FIG. 1

**FIG. 2**

**FIG. 3**

Overall

Postprandial

FIG. 4

EP 4 564 361 B1

FIG. 5

**FIG. 6**

FIG. 7

FIG. 8

**EP 4 564 361 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- AU 2013309425 A1